# EUROPEAN PATENT SPECIFICATION

(11) **EP 1 874 739 B1**
(45) Date of publication and mention of the grant of the patent: **26.05.2010**
(21) Application number: 06742609.8
(22) Date of filing: 19.04.2006
(51) Int. Cl.: C07D 243/08, C07D 401/12, C07D 401/14, C07D 403/12, C07D 405/12, A61K 31/5513

(54) **PHARMACEUTICALLY ACTIVE DIAZEPANES**
PHARMAZEUTISCH AKTIVE DIAZEPANE
DIAZEPANES PHARMACEUTIQUEMENT ACTIFS

(30) Priority: 19.04.2005 GB 0507918
(43) Date of publication of application: 09.01.2008
(60) Divisional application: 10155546.4
(73) Proprietor: Novartis AG, 4056 Basel (CH)
(72) Inventor: OBERHAUSER, Berndt, A-1230 Wien (AT); SCHOLZ, Dieter, A-1040 Wien (AT)
(74) Representative: Schaller, Hans
(86) International application number: PCT/EP2006/003583
(87) International publication number: WO 2006/111371

(56) References cited:
- WO-A-01/27102
- WO-A-2004/065382
- US-B1- 6 399 599
- WATTANASIN S ET AL: "1,4-Diazepane-2-ones as novel inhibitors of LFA-1" BIOORGANIC & MEDICINAL CHEMISTRY LETTERS, OXFORD, GB, vol. 13, no. 3, 9 September 2002 (2002-09-09), pages 499-502, XP002277292 ISSN: 0960-894X

## Description

The present invention relates to organic compounds, e.g. pharmaceutically active diazepanes.

Leukocyte Function-Associated Antigen One (LFA-1) is a cell adhesion molecule selectively expressed on leukocytes, and plays a major role in the activation and trafficking of T lymphocytes in the tissue at sites of inflammation. In the last decade a large body of preclinical data has accumulated to establish the importance of LFA-1 as a biological target, particularly in chronic, T-cell driven inflammatory conditions.

WO 2004/065382, WO01/27102, US6399599 and Wattanasin et al (Bioorg. Med Chem Letters, 2002, 13, p.499 ff) all disclose certain 1,4 Diazepan-2-ones and the LFA inhbiting properties.

Surprisingly a certain class of compounds are provided which mediate, e.g. inhibit, the activity of LFA-1, e.g. activity of LFA-1/ICAM-1, LFA-1/ICAM-2, LFA-1/ICAM-3 or LFA-1/JAM-1 interactions.

In one aspect the present invention provides a compound of formula 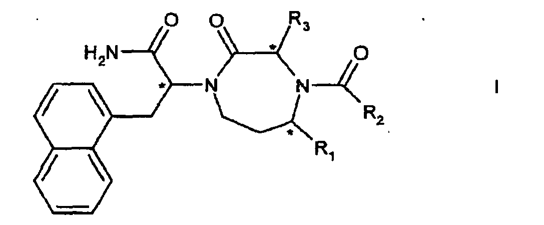 e.g. including a compound of formula 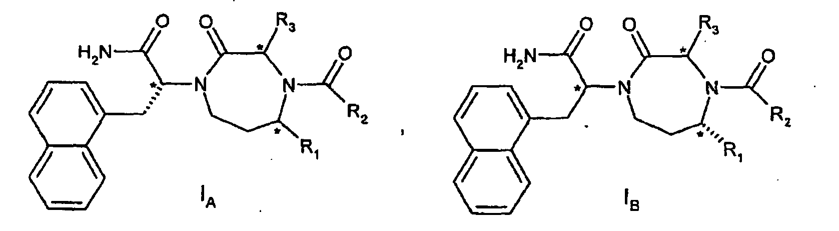 or of formula 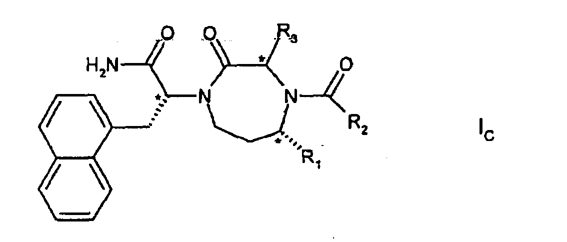 wherein
- R₁: is methyl;
- R₂: is pyridinylmethylaminocarbonylmethyl, optionally in the form of an N-oxide; and
- R₃: is substituted (C₆₋₁₈)aryl, e.g. one or morefold substituted, e.g. wherein substituents include (C₁₋₄)alkyl, (C₁₋₄)alkoxy halo(C₁₋₆)alkyl, halo(C₁₋₆)alkoxy, cyano,carboxy, hydroxy, amino, (C₁₋₆)alkyl- and (C₁₋₆)dialkylamino, (C₁₋₆)alkylcarbonylamino, (C₁₋₆)alkoxycarbonylamino, (C₁₋₈)alkoxycarbonyl, heterocyclyl, or halogen, preferably halogen.

In another aspect the present invention provides a compound of formula I, wherein R₃ is phenyl substituted by one or more, e.g. two, halogen, and R₂ and R₁ are as defined above.

In a compound of formula I each single defined substitutent may be a preferred substituent, e.g. independently of each other substitutent defined.

In another aspect the present invention provides 3-(C₆₋₁₂)Aryl-5-(C₁₋₄)alkyl-2-oxo-[1,4]diazepan-1-yl]-3-naphthalen-1-yl-propionamides, such as 3-(C₆)aryl-5-methyl-2-oxo-[1,4]diazepan-1-yl]-3-naphthalen-1-yl-propionamides, which are acylated in position 4.of the diazepane ring, wherein (C₆₋₁₂)aryl is substituted aryl, e.g. substituted as described in the meaning of R₃ above.

In another aspect the present invention provides a compound selected from the group consisting of the compounds as set out in Examples 1 to 65 (TABLE 1) below.

Compounds provided by the present invention are hereinafter designated as "compound(s) of (according to) the present invention". A compound of the present invention includes a compound in any form, e.g. in free form, in the form of a salt, in the form of a solvate and in the form of a salt and a solvate.

In another aspect the present invention provides a compound of the present invention in the form of a salt.

Such salts include preferably pharmaceutically acceptable salts, although pharmaceutically unacceptable salts are included, e.g. for preparation / isolation / purification purposes.

A salt of a compound of the present invention includes salts of a compound of formula I with an acid, e.g. trifluoroacetic acid, hydrochloric acid, or a trialylammonium salt, such as a trialkylammonium chloride salt.

A compound of the present invention in free form may be converted into a corresponding compound in the form of a salt; and vice versa. A compound of the present invention in free form or in the form of a salt and in the form of a solvate may be converted into a corresponding compound in free form or in the form of a salt in non-solvated form; and vice versa.

A compound of of the present invention may exist in the form of isomers and mixtures thereof; e.g. optical isomers, diastereoisomers, cis/trans conformers. A compound of the present invention may e.g. contain asymmetric carbon atoms and may thus exist in the form of enatiomers or diastereoisomers and mixtures thereof, e.g. racemates. A compound of the present invention may may be present in the (R)-, (S)- or (R,S)-configuration preferably in the (R)- or (S)-configuration, regarding specified positions in the compound of the present invention. A compound provided by the present invention may be in the (R)- and in the (S)-configuration, e.g. including mixtures thereof, regarding the subsituent in position indicated with a star in a compound of formula I, and is preferably in the (R)- or in the (S)-configuration. A compound of the present invention may be also in the form of cis or trans conformation, e.g. if R₁ is alkenyl.

Isomeric mixtures may be separated as appropriate, e.g. according, e.g. analogously, to a method as conventional, to obtain pure isomers. The present invention includes a compound of the present invention in any isomeric form and in any isomeric mixture.
The present invention also includes tautomers of a compound of the present invention, where tautomers can exist.

In another aspect the present invention provides a process for the preparation of a compound of the present invention comprising acylating a compound of formula 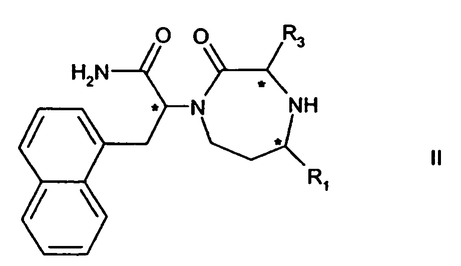 wherein R, and R₃ are as defined above,
e.g. reacting with a compound of formula 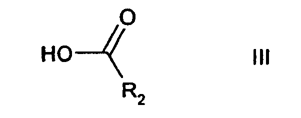 or with a compound of formula 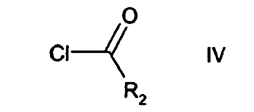 wherein R₂ is as defined above,
in the presence of a condensing agent, e.g. a carbodiimide, and a base, e.g. an amine, such as diisopropylethylamine or dimethylaminopyridine, in organic solvent, e.g. polar organic solvent, such as N,N-dimethylformamide, and isolating a compound of formula I, wherein R₁, R₂ and R₃ are as defined above, from the reaction mixture.

In an intermediate of formula II, III or IV (starting materials), functional groups, if present, optionally may be in protected form or in the form of a salt, if a salt-forming group is present. Protecting groups, optionally present, may be removed at an appropriate stage, e.g. according, e.g. analogously, to a method as conventional.
A compound of formula I thus obtained may be converted into another compound of formula I, e.g. or a compound of formula I obtained in free form may be converted into a salt of a compound of formula I and vice versa.

An optionally protected group R₂ of formula III or IV e.g. includes phenyl substituted by an amine. Such amine may be protected by an appropriate protection group, e.g. including tert-butoxycarbonyl (Boc), which protecting group may be removed after reaction of a compound of formula II with a compound of formula III to obtain a free amine group. Conversion into another compound of formula I e.g. includes alkylating or acylating such amine group as appropriate, e.g. according, e.g. analogously, to a method as conventional, or as specified herein.

3-(C₆₋₁₂)Aryl-5-(C₁₋₄)alkyl-2-oxo-[1,4]diazepan-1-yl]-3-naphthalen-1-yl-propionamides, such as 3-phenyl-5-methyl-2-oxo-[1,4]diazepan-1-yl]-3-naphthalen-1-yl-propionamides, which are acylated in position 4.of the diazepane ring may be prepared by acylation of the nitrogen atom in position 4 of 3-(C₆₋₁₂)Aryl-5-(C₁₋₄)alkyl-2-oxo-[1,4]diazepan-1-yl]-3-naphthalen-1-yl-propionamides, such as 3-phenyl-5-methyl-2-oxo-[1,4]diazepan-1-yl]-3-naphthalen-1-yl-propionamides, by a method as appropriate, e.g. according, e.g. analogously, to a method as conventional, or as specified herein.

A general procedure for the synthesis of compounds of the present invention including the synthesis of starting materials is outlined below in procedures A1 to A8.

### Procedure A1. Synthesis of a ketal intermediate

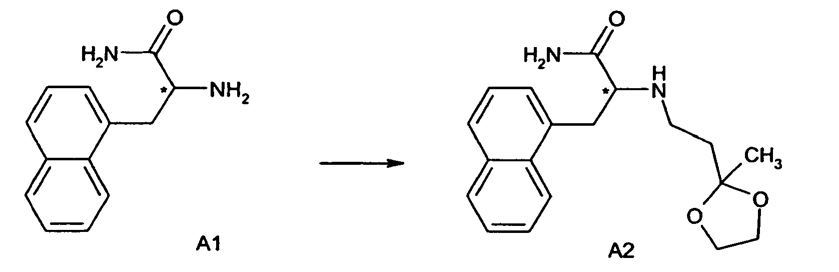

Naphthylalanine amide of formula A1 and 0.3 equiv. of N-methyl-morpholine are dissolved in dioxane and 1.5 equiv. of methylvinylketone are added. The mixture obtained is stirred at RT for 15 hours and 5 equiv. of 2-methoxydioxolane and 1.5 equiv. of TsOH-monohydrate are added. The mixture obtained is stirred and diluted with EtAc. The organic phase obtained is washed and dried, solvent is evaporated and a ketal intermediate of formula A2 is obtained, which is optionally purified or used without further purification.

### Procedure A2. Amine-protection of (C₆₋₁₈)arylglycines

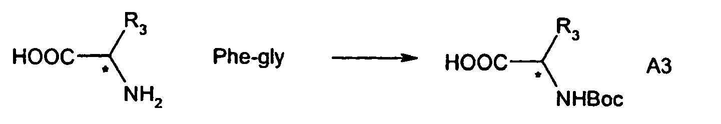

A compound of formula Phe-gly, wherein R₃ is as defined above, is dissolved in MeOH. 2 equiv. of NaHCO₃ and 1.2 equiv. of Boc-anhydride are added to the solution obtained and the suspension obtained is heated at 50° under stirring. From the mixture obtained solvent is evaporated and H₂O and toluene are added. The phases obtained are separated and the organic phase obtained is extracted with 1 N NaOH. The pH of the aqueous phase obtained is adjusted to pH 3 and the mixture obtained is extracted with EtAc. The organic phase obtained is dried, solvent is evaporated and racemic Boc-phenylglycine of formula A3, wherein R₃ is as defined above, is obtained.

### Procedure A3. Amine acylation

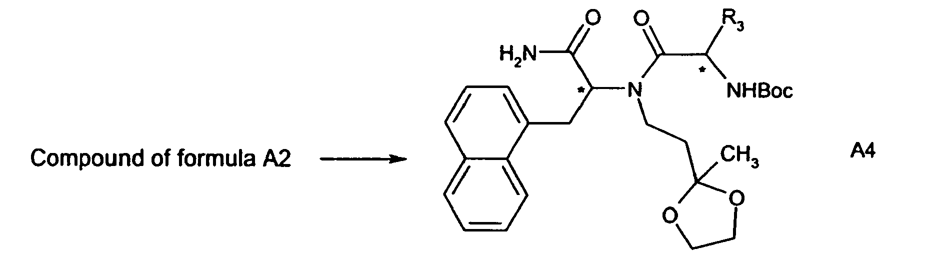

1 equiv. of a compound of formula A2, wherein R₃ is as defined above, 1.5 equiv. of racemic (substituted) Boc-phenylglycine of formula A3, wherein R₃ has the meaning as defined above, and 0.12 equiv. of 1-hydroxy-7-azabenzotriazole are dissolved in DMF. 1.5 equiv. of diisopropylethylamine and 1.5 equiv. of EDC in free base form are added during 15 hours at rt. Solvent is evaporated, the evaporation residue obtained is diluted with EtAc and extracted with 1 N HCl and 5% NaHCO₃ solution. The organic phase obtained is dried and solvent is evaporated. A compound of formula A4, wherein R₃ is as defined above, is obtained in the form of a diastereoisomeric mixture.

### Procedure A4. Deprotection and reductive ring closure

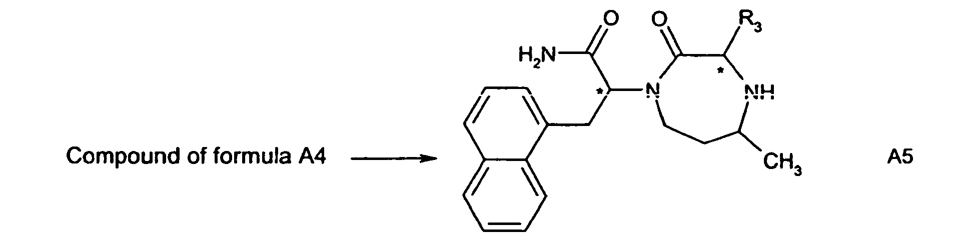

A compound of formula A4, wherein R₃ is as defined above, is dissolved in TFA/H₂O at 0°. The mixture obtained is stirred, quenched with H₂O and solvent is evaporated. A diastereoisomeric mixture of a compound of formula A4, wherein the Boc-NH- group is deprotected is obtained and is dissolved in MeOH/H₂O. The pH of the mixture obtained is adjusted to pH 5. 0.5 equiv. of a NaCNBH₃-solution in MeOH/H₂O are added at 0° to the mixture obtained, the mixture obtained is stirred, solvent is evaporated and the evaporation residue obtained is diluted with EtAc.3.5M phosphate-buffer of pH 4 are added to the mixture obtained, two phases are obtained and are separated. The organic phase obtained is extracted with 5% NaHCO₃ solution, dried and solvent is evaporated. A compound of formula A5, wherein R₃ is as defined above, is obtained.

In a specific embodiment a of the present invention a compound of formula I wherein R₁ and R₃ are as described above and R₂ is (C₁₋₄)alkyl substituted by amino may be obtained according to Procedure A5:

### Procedure A5. Chloro-(C₁₋₄)alkylcarbonylchloride derivative (ALK = (C₁₋₄)alkyl)

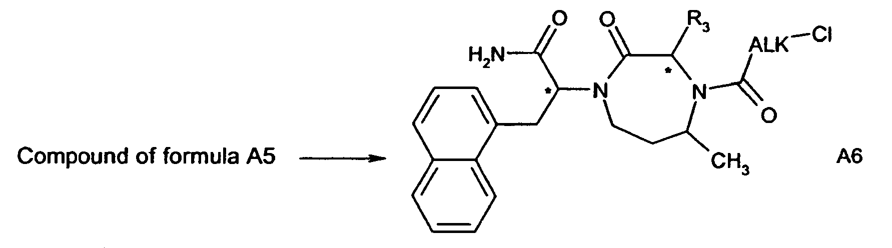

Chloro-(C₁₋₄)alkylcarbonylchloride is slowly added to a solution of a compound of formula A5 and diisopropylamine in CH₂Cl₂ at 0°. After 4 hours the mixture obtained is diluted with ethyl acetate and extracted with 5% aq. NaHCO₃ solution and 1 N aq. HCl. Solvent is evaporated and a compound of formula A6 (mixture of diastereomeres) is obtained.

### Procedure A6. Azido-acetyl derivative

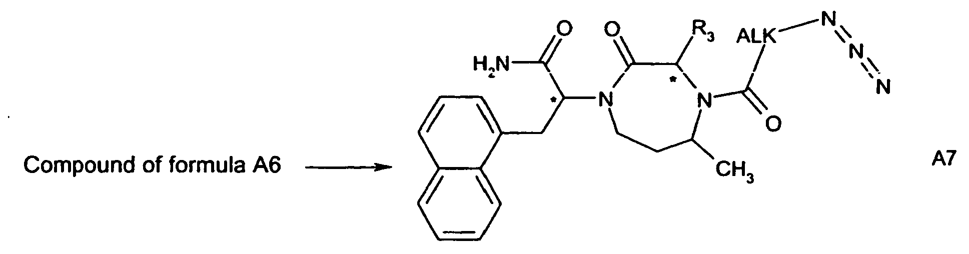

A solution a compound of formula A6 and 2 equiv. NaN₃ is stirred in DMF at 60° for 4 hours and solvent is evaporated, the evaporation residue obtained is diluted with EtAc and the mixture obtained is extracted with 1 N aq.HCl and 5% aq. NaHCO₃ solution. After drying solvent is evaporated and the residual solid obtained is subjected to chromatography. A compound of formula A7 is obtained.

### Procedure A7. Amino-(C₁₋₄)alkylcarbonyl derivatives

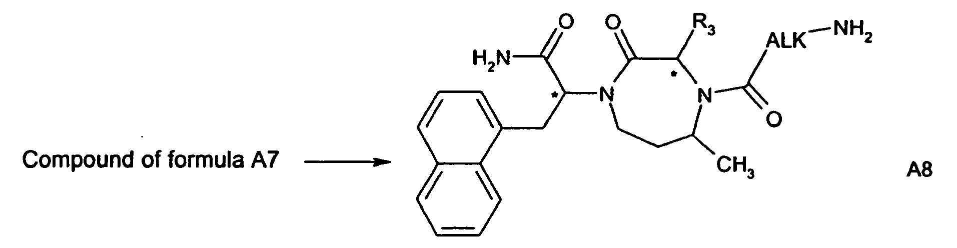

A solution of a compound of formula A7 in MeOH/1N HCl 10:1 is hydrogenated with Pd/C at rt (1 atm) for 48 hours. Catalyst is removed, solvent is evaporated and a compound of formula A8, e.g. in the form of a hydrochloride, is obtained.

A compound of formula A8 may be alkylated, acylated or sulfonylated as appropriate, e.g. analogously to a method as conventional, or as specified herein.

In another aspect the present invention provides
- a compound of formula II wherein R₁ and R₃ are as defined above,
- a compound of formula A6, wherein R₁ and R₂ are defined as above,
- a compound of formula A7 wherein R₁ and R₃ are as defined above, and
- a compound of formula A8 wherein R₁ and R₃ are as defined above,
e.g. in the form of a salt,
e.g. useful as intermediates for the production of a compound of the present invention.

Any compound described herein, e.g. a compound of the present invention and intermediates of formula II,III, IV, A1, A2, A3, A4, A5, A6, A7 and A8 may be prepared as appropriate, e.g. according, e.g. analogously, to a method as conventional, e.g. or as specified herein.

The compounds of the present invention, e.g. including a compound of formula I, exhibit pharmacological activity and are therefore useful as pharmaceuticals, e.g. useful for therapy. E.g., the compounds of the present invention are found to inhibt LFA-1 activity. e.g. by mediating, such as inhibiting, the activity of LFA-1/ICAM-1, LFA-1/ICAM-2, LFA-1/ICAM-3 or LFA-1/JAM-1 interactions and thus mediating disorders wherein LFA-1 plays a causal or contributory role. Such activity may be determined, e.g. as indicated in in vitro and in vivo TEST SYSTEMS herein.

### A. In vitro TEST SYSTEM (Cell free assay)

The assay determines the binding of soluble human ICAM-1 to immobilized human LFA-1. LFA-1 is purified from JY cells, a human lymphoblastoid B cell-line, by immunoaffinity chromatography analogously as described by Dustin et al., J. Immunol. 148, 2654-2663, 1992. ICAM-1 mouse Cκ fusion protein (ICAM-1) is produced using the baculovirus system as described by Weitz-Schmidt et al., Anal. Biochem. 238,184-190, 1996.
Purified LFA-1 is diluted 1:20 in phosphate buffered saline (PBS) containing 2 mM MgCl₂, pH 7.4 and coated onto microtiter plates (Nunc) at 37° for 3 hours. Plates are blocked with 1% heat-treated bovine serum albumin in PBS for 2 hours at 37° followed by a washing step using PBS, 2 mM MgCl₂, 1% fetal calf serum, pH 7.4 (assay buffer). Compounds of the present invention (10 mM solution in DMSO) are diluted in assay buffer and added to the plates. Biotinylated recombinant ICAM-1 in assay buffer (6 µg/ml) is added and allowed to bind at 37° for one hour. After incubation, wells are washed with assay buffer. Streptavidin-peroxidase diluted 1:5000 in assay buffer is added and incubated for 45 min at 37°. Plates are washed with assay buffer and 2,2'-azino-bis(3-ethylbenzothiazoline-6-sulfonic acid) diammonium salt substrate solution is added to each well. The reaction is stopped after 20 minutes and bound ICAM-1 is determined by measuring the optical density at 405 nm in a microplate reader.
In this assay the compounds of the present invention exhibit activity, e.g. the compounds of the present invention inhibit adhesion of LFA-1 to ICAM-1 with an IC₅₀ in the nanomolar up to the low micromolar range. The compounds of examples 13 and 14 are preferred compounds of the present invention and show IC₅₀ values of 0.43 or 0.09 µM, respectively, in this assay. It was surprisingly found that compounds of formula I, wherein R₃ is substituted phenyl show better IC₅₀ values in such LFA-1 in vitro TEST SYSTEM than compounds of formula I, wherein R₃ is unsubstituted phenyl.

### B. In vivo TEST SYSTEM Allergic Contact Dermatitis (ACD)

Groups of 8 female NMRI mice are sensitized on the shaved abdomen with 50 µl of oxazolone (2% in acetone) and challenged with 10 µl of 0.2% oxazolone on the inner surface of the right ear 7 days later. The unchallenged left ears serve as normal controls and dermatitis is evaluated from the individual differences in auricular weights, which are taken as a measure of inflammatory swelling 24 hours after the challenge. The test groups are treated with the test compounds orally (2 hours after challenge), the controls are treated similarly with the vehicles alone. For oral administration the compounds are administered in an oil in H₂O emulsion. Dermatitis is evaluated in test- and control groups. The animals are killed and both ears are cut off and weighed. Inhibitory activity of the test compounds is calculated from the differences in right and left ears (internal controls) in mice treated with test compounds compared with animals treated with the vehicle only. The data of the test-and the vehicle-treated control groups are statistically analyzed by ANOVA followed by Dunnet T-test (normal distribution or data) or by H and U-test, respectively. When administered p.o. at a dose of from 0.03 to 20 mg/kg, the compounds of the present invention inhibit the elicitation phase of allergic contact dermatitis based on the differences in auricular weights.

The compounds of the present invention show activity in the above described assays and are therfore indicated for the treatment of disorders (diseases) mediated by LFA-1 activity with its ligands involved in cell adhesion, migration and activation.

Disorders, e.g. including diseases, mediated byLFA-1 activity and which are prone to be successfully treated with LFA-1 antagonists, e.g. with a compound of the present invention, include disorders, wherein the activity of LFA-1 plays a causal or contributory role.

The compounds of the present invention may be preferably useful for treatment of inflammatory disorders, allergic disorders, autoimmune disorders or cancer, more preferably inflammatory disorders, allergic disorders, autoimmune disorders, such as inflammatory disorders.

Disorders mediated by LFA-1 e.g. include
- disorders associated with inflammation
- e.g. including (chronic) inflammatory disorders, disorders related with the inflammation of the bronchi, e.g. including bronchitis, cervix, e.g. including cervicitis, conjunctiva, e.g. conjunctivitis, esophagus, e.g. esophagitis, heart muscle, e.g. myocarditis, rectum, e.g. proctitis, sclera, e.g. scleritis, gums, involving bone, pulmonary inflammation (alveolitis), airways, e.g. asthma, such as bronchial asthma, acute respiratory distress syndrome (ARDS), inflammatory skin disorders such as contact hypersensitivity, atopic dermatitis; fibrotic disease (e.g., pulmonary fibrosis), encephilitis, inflammatory osteolysis,
- **disorders associated with conditions of the immune system**,
   immune, such as autoimmune disorders e.g. including Graves' disease, Hashimoto's disease (chronic thyroiditis), multiple sclerosis, rheumatoid arthritis, arthritis, gout, , osteoarthritis, scleroderma, lupus syndromes, systemic lupus erytomatosis, Sjgren's syndrome, psoriasis, inflammatory bowel disease, including Crohn's disease, colitis, e.g. ulcerative colitis; sepsis, septic shock, autoimmune hemolytic anemia (AHA), autoantibody triggered urticaria, pemphigus, nephritis, glomerulonephritis, Goodpastur syndrom, ankylosing spondylitis, Reiter's syndrome, polymyositis, dermatomyositis, cytokine-mediated toxicity, interleukin-2 toxicity, alopecia areata, uveitis, lichen planus, bullous pemphigoid, epidermolysis bullosa, myasthenia gravis.
- **disorders associated with allergic conditions,**
   e.g. including delayed-type hypersensitivity, allergic conjunctivitis, drug allergies, rhinitis, allergic rhinitis, vasculitis, contact dermatits;

A compound of the present invention may be preferably useful for treatment of psoriasis, rheumatoid arthritis, inflammatory bowel diseases (Crohn's disease, ulcerative colitis), (systemic) lupus erythematosus, atopic dermatitris, Sjogren' syndrom, rejection after transplantation and graft vs. host disease. In one preferred aspect a compound of the present invention may be useful in the treatment of autoimmune diseases, e.g. rheumatoid arthritis, inflammatory bowel disease, or inflammatory diseases, e.g. psoriasis or atopic dermatitis.

In another aspect the present invention provides
- a compound of the present invention for use as a pharmaceutical,
- the use of a compound of the present invention as a pharmaceutical
e.g. for the treatment of disorders mediated by IFA-1 activity.

For pharmaceutical use one or more compounds of the present invention may be used, e.g. one, or a combination of two or more compounds of the present invention, preferably one compound of the present invention is used.

A compound of the present invention may be used as a pharmaceutical in the form of a pharmaceutical composition.

In another aspect the present invention provides a pharmaceutical composition comprising a compound of the present invention in association with at least one pharmaceutically acceptable excipient, e.g. appropriate carrier and/or diluent, e.g. including fillers, binders, disintegrants, flow conditioners, lubricants, sugars or sweeteners, fragrances, preservatives, stabilizers, wetting agents and/or emulsifiers, solubilizers, salts for regulating osmotic pressure and/or buffers.

In another aspect the present invention provides
- a pharmaceutical composition of the present invention for use of treating disoders which are mediated by LFA-1 activity.
- the use of a pharmaceutical composition of the present invention for treating disorders which are by LFA-1 activity.

In a further aspect the present invention provides a method of treating disorders which are mediated by LFA-1 activity, e.g. including disorders as specified above, which treatment comprises administering to a subject in need of such treatment an effective amount of a compound of the present invention; e.g. in the form of a pharmaceutical composition.

In another aspect the present invention provides
- a compound of the present invention for the manufacture of a medicament,
- the use of a compound of the present invention for the manufacture of a medicament, e.g. a pharmaceutical composition,
for the treatment of disorders, which are mediated by LFA-1 activity.

Treatment includes treatment and prophylaxis (prevention).
For such treatment, the appropriate dosage will, of course, vary depending upon, for example, the chemical nature and the pharmakokinetic data of a compound of the present invention used, the individual host, the mode of administration and the nature and severity of the conditions being treated. However, in general, for satisfactory results in larger mammals, for example humans, an indicated daily dosage includes a range
- from about 0.001 g to about 1.5 g, such as 0.001 g to 1.5 g;
- from about 0.01 mg/kg body weight to about 20 mg/kg body weight, such as 0.01 mg/kg body weight to 20 mg/kg body weight,
for example administered in divided doses up to four times a day.

A compound of the present invention may be administered to larger mammals, for example humans, by similar modes of administration than conventionally used with other mediators, e.g. low molecular weight inhibitors of IFA-1 activity.

A compound of the present invention may be administered by any conventional route, for example enterally, e.g. including nasal, buccal, rectal, oral, administration; parenterally, e.g. including intravenous, intramuscular, subcutanous administration; or topically; e.g. including epicutaneous, intranasal, intratracheal administration; via medical devices for local delivery, e.g. stents,
e.g. in form of coated or uncoated tablets, capsules, (injectable) solutions, solid solutions, suspensions, dispersions, solid dispersions; e.g. in the form of ampoules, vials, in the form of creams, gels, pastes, inhaler powder, foams, tinctures, lip sticks, drops, sprays, or in the form of suppositories.
For topical use, e.g. including administration to the eye, satisfactory results may be obtained with local administration of a 0.5-10 %, such as 1-3% concentration of active substance, e.g. several times daily, e.g. 2 to 5 times daily.

The compounds of the present invention may be administered in the form of a pharmaceutically acceptable salt, or in free form; optionally in the form of a solvate. A compound of the present invention in the form of a salt and/or in the form of a solvate exhibit the same order of activity as a compound of the present invention in free form.

A compound of the present invention may be used for any method or use as described herein alone or in combination with one or more, at least one, other, second drug substance.

In another aspect the present invention provides
- A combination of a compound of the present invention with at least one second drug substance;
- A pharmaceutical combination comprising a compound of the present invention in combination with with at least one second drug substance;
- A pharmaceutical composition comprising a compound of the present invention in combination with with at least one second drug substance and one or more pharmaceutically acceptable excipient(s).;
- A compound of the present invention in combination with at least one second drug substance, e.g. in the form of a pharmaceutical combination or composition, for use in any method as defined herein, e.g.
   - A combination, a pharmaceutical combination or a pharmaceutical composition, comprising a compound of the present invention and at least one second drug substance for use as a pharmaceutical;
   - The use as a pharmaceutical of a compound of the present invention in combination with at least one second drug substance, e.g. in the form of a pharmaceutical combination or composistion;
- A method for treating disordes mediated by IFA-1 activity in a subject in need thereof, comprising co-administering, concomitantly or in sequence, a therapeutically effective amount of a compound of the present invention and at least one second drug substance, e.g. in the form of a pharmaceutical combination or composition;
- A compound of the present invention in combination with at least one second drug substance, e.g. in the form of a pharmaceutical combination or composition, for use in the preparation of a medicament for use in disorders mediated by IFA-1 activity.

Combinations include fixed combinations, in which a compound of the present invention and at least one second drug substance are in the same formulation; kits, in which a compound of the present invention and at least one second drug substance in separate formulations are provided in the same package, e.g. with instruction for co-administration; and free combinations in which a compound of the present invention and at least one second drug substance are packaged separately, but instruction for concomitant or sequential administration are given.

In another aspect the present invention provides
- A pharmaceutical package comprising a first drug substance which is a compound of the present invention and at least one second drug substance, beside instructions for combined administration;
- A pharmaceutical package comprising a compound of the present invention beside instructions for combined administration with at least one second drug substance;
- A pharmaceutical package comprising at least one second drug substance beside instructions for combined administration with a compound of the present invention.

Treatment with combinations according to the present invention may provide improvements compared with single treatment.

In another aspect the present invention provides
- A pharmaceutical combination comprising an amount of a compound of the present invention and an amount of a second drug substance, wherein the amounts are appropriate to produce a synergistic therapeutic effect;
- A method for improving the therapeutic utility of a compound of the present invention comprising co-administering, e.g. concomitantly or in sequence, of a therapeutically effective amount of a compound of the present invention and a second drug substance.
- A method for improving the therapeutic utility of a second drug substance comprising co-administering, e.g. concomitantly or in sequence, of a therapeutically effective amount of a compound of the present invention and a second drug substance.

A combination of the present invention and a second drug substance as a combination partner may be administered by any conventional route, for example as set out above for a compound of the present invention. A second drug may be administered in dosages as appropriate, e.g. in dosage ranges which are similar to those used for single treatment, or, e.g. in case of synergy, even below conventional dosage ranges.

Pharmaceutical compositions according to the present invention may be manufactured according, e.g. analogously, to a method as conventional, e.g. by mixing, granulating, coating, dissolving or lyophilizing processes. Unit dosage forms may contain, for example, from about 0.1 mg to about 1500 mg, such as 1 mg to about 1000 mg. Pharmaceutical compositions comprising a combination of the present invention and pharmaceutical compositions comprising a second drug as described herein, may be provided as appropriate, e.g. according, e.g. analogously, to a method as conventional, or as described herein for a pharmaceutical composition of the present invention.

By the term "second drug substance" is meant a chemotherapeutic drug, especially any chemotherapeutic agent other than a compound of the present invention, such as a compound of formula I.
For example, a second drug substance as used herein includes e.g.
anti-inflammatory and/or immunomodulatory drugs, anticancer drugs, antiallergic drugs, anesthetic drugs

Anti-inflammatory and/or immunomodulatory drugs which are prone to be useful in combination with a compound of the present invention include e.g.
- mediators, e.g. inhibitors of mTOR activity, including rapamycins, e.g. rapamycin of formula 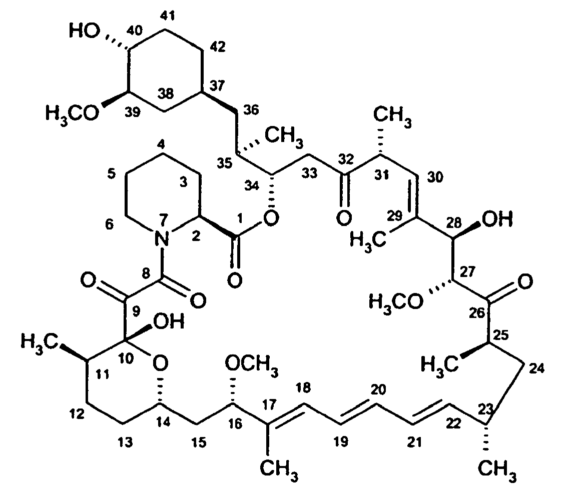 40-O-(2-hydroxyethyl)-rapamycin, 32-deoxorapamycin, 16-O-substituted rapamycins such as 16-pent-2-ynyloxy-32-deoxorapamycin, 16-pent-2-ynyloxy-32 (S or R) -dihydro-rapamycin, 16-pent-2-ynyloxy-32(S or R)-dihydro-40-O-(2-hydroxyethyl)-rapamycin, 40-[3-hydroxy-2-(hydroxy-methyl)-2-methylpropanoate]-rapamycin (also known as CCl779), 40-epi-(tetrazolyl)-rapamycin (also known as ABT578), the so-called rapalogs, e. g. as disclosed in W09802441, WO0114387 and WO0364383, such as AP23573, and compounds disclosed under the name TAFA-93 and biolimus (biolimus A9);
- mediators, e.g. inhibitors, of calcineurin, e.g. cyclosporin A, FK 506;
- ascomycins having immuno-suppressive properties, e.g. ABT-281, ASM981;
- corticosteroids; cyclophosphamide; azathioprene; leflunomide; mizoribine;
- mycophenolic acid or salt; mycophenolate mofetil;
- 15-deoxyspergualine or an immunosuppressive homologue, analogue or derivative thereof;
- mediators, e.g. inhibitors, of bcr-abl tyrosine kinase activity;
- mediators, e.g. inhibitors, of c-kit receptor tyrosine kinase activity;
- mediators, e.g. inhibitors, of PDGF receptor tyrosine kinase activity, e.g. Gleevec (imatinib);
- mediators, e.g. inhibitors, of p38 MAP kinase activity,
- mediators, e.g. inhibitors, of VEGF receptor tyrosine kinase activity,
- mediators, e.g. inhibitors, of PKC activity, e.g. as disclosed in W00238561 or WO0382859, e.g. the compound of Example 56 or 70;
- mediators, e.g. inhibitors, of JAK3 kinase activity, e.g. N-benzyl-3,4-dihydroxy-benzylidene-cyanoacetamide α-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-211, 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550), or a compound as disclosed in WO2004052359 or W02005066156;
- mediators, e.g. agonists or modulators of S1P receptor activity, e.g. FTY720 optionally phosphorylated or an analog thereof, e.g. 2-amino-2-[4-(3-benzyloxyphenylthio)-2-chlorophenyl]ethyl-1,3-propanediol optionally phosphorylated or 1-{4-[1-(4-cyclohexyl-3-trifluoromethyl-benzyloxyimino)-ethyl]-2-ethyl-benzyl}-azetidine-3-carboxylic acid or its pharmaceutically acceptable salts;
- immunosuppressive monoclonal antibodies, e.g., monoclonal antibodies to leukocyte receptors, e.g., Blys/BAFF receptor, MHC, CD2, CD3, CD4, CD7, CD8, CD25, CD28, CD40, CD45, CD52, CD58, CD80, CD86, IL-12 receptor, IL-17 receptor, IL-23 receptor or their ligands;
- other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4Ig (for ex. designated ATCC 68629) or a mutant thereof, e.g. LEA29Y;
- mediators, e.g. inhibitors of adhesion molecule activities, e.g. other LFA-1 antagonists, ICAM-1 or -3 antagonists than those provided by the present invention, VCAM-4 antagonists or VLA-4 antagonists,
- mediators, e.g. antagonists of CCR9 acitiviy,
- mediators, e.g. inhibitors, of MIF activity,
- 5-aminosalicylate (5-ASA) agents, such as sulfasalazine, Azulfidine®, Asacol®, Dipentum®, Pentasa®, Rowasa®, Canasa®, Cotazal®, e.g. drugs containing mesalamine; e.g mesalazine in combination with heparin;
- mediators, e.g. inhibitors, of TNF-alpha activity, e.g. including antibodies which bind to TNF-alpha, e.g. infliximab (Remicade®),
- nitric oxide releasing non-steriodal anti-inlammatory drugs (NSAIDs), e.g. including COX-inhibiting NO-donating drugs (CINOD);
- phospordiesterase, e.g. mediatorssuch as inhibitors of PDE4B activity,
- mediators, e.g. inhibitors, of caspase activity,
- mediators, e.g. agonists, of the G protein coupled receptor GPBAR1,
- mediators, e.g. inhibitors, of ceramide kinase activity,
- 'multi-functional anti-inflammatory' drugs (MFAIDs), e.g. cytosolic phoshpholipase A2 (cPLA2) inhibitors, such as membrane-anchored phospholipase A2 inhibitors linked to glycosaminoglycans;
- antibiotics, such as penicillins, cephalosporins, erythromycins, tetracyclines, sulfonamides, such as sulfadiazine, sulfisoxazole; sulfones, such as dapsone; pleuromutilins, fluoroquinolones, e.g. metronidazole, quinolones such as ciprofloxacin; levofloxacin; probiotics and commensal bacteria e.g. Lactobacillus, Lactobacillus reuteri;
- antiviral drugs, such as ribivirin, vidarabine, acyclovir, ganciclovir, zanamivir, oseltamivir phosphate, famciclovir, atazanavir, amantadine, didanosine, efavirenz, foscarnet, indinavir, lamivudine, nelfinavir, ritonavir, saquinavir, stavudine, valacyclovir, valganciclovir, zidovudine;.

Anticancer drugs which are prone to be useful as a combination partner with a compound of the present invention e.g. include mediators, such as inhibitors of catechol-O-methyltransferase, e.g. entacapone, mediators, e.g. inhibitors, of gonadotropin-releasing (LH-RH) hormone analogs, e.g. leuprolide, ispinesib, oxaliplatin, triciribine, permetrexed (Alimta®), sunitinib (SU11248), temozolidine, daunorubicin, dactinomycin, doxorubicin, bleomycin, mitomycin, nitrogen mustard, chlorambucil, melphalan, cyclophosphamide, 6-mercaptopurine, 6-thioguanine, cytarabine (CA), 5-fluorouracil(5-FU),floxuridine (5-FUdR), methotrexate (MTX), colchicine, vincristine, vinblastine, etoposide, teniposide, cisplatin, diethylstilbestrol (DES), tipifarnib, bortezomib and drugs such as disclosed as "chemotherpeutic agents" in WO02066019, e.g. on pages 5 and 6 under i) to x), in more detail on pages 6 to 11, namely agents which are disclosed to be useful in combination treatment of solid tumors. WO02066019 is introduced herein by reference.
In cancer therapy, naturally any treatment according to the present invention can be combinded with radiotherapy:

Anesthetics which are prone to be useful as a combination partner with a compound of the present invention e.g. include ethanol, bupivacaine, chloroprocaine, levobupivacaine, lidocaine, mepivacaine, procaine, ropivacaine, tetracaine, desflurane, isoflurane, ketamine, propofol; sevoflurane, codeine, fentanyl, hydromorphone, marcaine, meperidine, methadone, morphine, oxycodone, remifentanil, sufentanil, butorphanol, nalbuphine, tramadol, benzocaine, dibucaine, ethyl chloride, xylocaine, and phenazopyridine.

If the compounds of the present invention are administered in combination with other drugs dosages of the co-administered second drug will of course vary depending on the type of co-drug employed, on the specific drug employed, on the condition being treated, as in case of a compound of the present invention. In general dosages similar than those as provided by the second drug supplier may be appropriate

In the following examples and general procedures above all temperatures are in degree (°) Celsius. In the reaction schemes and corresponding description R₂ and R₃ are as defined above.

The following ABBREVIATIONS are used:
- AcOH: acetic acid
- Boc: tert-butoxy-carbonyl
- BuOH: n-butylalcohol
- CDI: carbodiimide
- DBU: 1,4-diaza-bicyclo[5.4.0]undec-7-en
- DIEA: diisopropylethylamine
- DIPCl: diisopropylcarbodiimide
- DMAP: N,N-dimethyl-4-aminopyridine
- DMF: N,N-dimethylformamide
- EDC: N-ethyl-N'-(3-dimethylaminopropyl)-carbodiimide
- EDC-HCl: N-ethyl-N'-(3-dimethylaminopropyl)-carbodiimide in the form of a hydrochloride
- EtAc: ethyl acetate
- equiv.: equivalent
- EX: Example
- HOAt: 1-hydroxy-7-azabenzotriazole i-PrOH isopropanol
- MeOH: methanol
- NaAc: sodium acetate
- NMM: N-methyl-morpholine
- n-Bu: n-butyl
- rt: room temperature
- THF: tetrahydrofurane
- TFA: trifluoroacetic acid
- Toi: toluene
- TsOH: p-toluenesulfonic acid
- Z: benzyloxycarbonyl

### EXAMPLES

### EXAMPLE 1

### 2-[3-(3-Chloro-phenyl)-5-methyl-4-(2-methylamino-acetyl)-2-oxo-[1,4]diazepan-1-yl]-3-naphthalen-1-yl-propionamide

A solu tion of 2-{[3-(3-Chloro-phenyl)-4-amino-methoxy-5-methyl]-2-oxo-[1,4]diazepan-1-yl}-3-naphthalen-1-yl-propionamide and an excess of benzaldehyde in MeOH is adjusted to pH 4 with acetic acid and Et₃N and treated with NaCNBH₃ at rt. After 1 hour, formalin solution and additional NaCNBH₃ are added. The reaction mixture obtained is diluted with EtAc, extracted with 0.2N phosphate buffer pH=8 and solvent is evaporated. The evaporation residue obtained is treated with MeOH/H₂O, acidified with 1 N aqeous HCl and hydrogenated at rt and 1 bar for 20 hours with Pd/C as catalyst. The catalyst is filtered off, solvent is evaporated and the evaporation residue obtained is subjected to chromatography. (R)-2-[(3S,5R)-3-(3-Chloro-phenyl)-5-methyl-4-(2-methylamino-acetyl)-2-oxo-[1,4]diazepan-1-yl]-3-naphthalen-1-yl-propionamide in the form of a trifluoroacetate is obtained.

### EXAMPLE 2

### N-{2-[4-(1-Carbamoyl-2-naphthalen-1-yl-ethyl)-2-(3-chloro-phenyl)-7-methyl-3-oxo-[1,4]diazepan-1-yl]-2-oxo-ethyl}-N-methyl-nicotinamide

2-[3-(3-Chloro-phenyl)-5-methyl-4-(2-methylamino-acetyl)-2-oxo-[1,4]diazepan-1-yl]-3-naphthalen-1-yl-propionamide (Example 1) is reacted with nicotinic acid in TFA and DMF in the presence of CDI.

N-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethyl}-N-methyl-nictotinamide in the form of a hydrochloride is obtained.

### EXAMPLE 3

### 2-[3-(3-Chloro-phenyl)-4-(3-dibutylamino-propionyl)-5-methyl-2-oxo-[1,4]diazepan-1-yl]-3-naphthalen-1-yl-propionamide

147 mg of the compound of example 56 as described below are dissolved in 3 ml of THF, 38 mg of Ytterbium (III) triflate and 1.5 mmol of the amine are added. The mixture obtained is stirred, diluted with CH₂Cl₂, extracted with 1 N HCl, dried, filtered, etheric HCl is added and solvent is evaporated.

(R)-2-[3-(3-Chloro-phenyl)-4-(3-dibutylamino-propionyl)-5-methyf-2-oxo-[1,4]diazepan-1-yl]-3-naphthalen-1-yl-propionamide in the form of a hydrochloride is obtained.

### EXAMPLES 4 to 8

### 2-[3-(3-Chloro-phenyl)-4-(3-cyclohexylamino-propionyl)-5-methyl-2-oxo-[1,4]diazepan-1-yl]-3-naphthalen-1-yl-propionamide

**2-[4-[3-(1-Benzyl-2-hydroxy-ethylamino)-propionyl]-3-(3-chloro-phenyl)-5-methyl-2-oxo-[1,4]diazepan-1-yl]-3-naphthalen-1-yl-propionamide,**
**2-[3-(3-Chloro-phenyl)-4-[3-(2-hydroxy-1-methyl-2-phenyl-ethylamino)-propionyl]-5-methyl-2-oxo-[1,4]diazepan-1-yl]-3-naphthalen-1-yl-propionamide, and**
**2-(3-(3-Chloro-phenyl)-4-{3-[(2-hydroxy-1-methyl-2-phenyl-ethyl)-methyl-amino]-propionyl}-5-methyl-2-oxo-[1,4]diazepan-1-yl]-3-naphthalen-1-yl-propionamide** Analogously to the method as described for example 3, but using appropriate starting materials, the compounds
(R)-2-[3-(3-Chloro-phenyl)-4-(3-cyclohexylamino-propionyl)-5-methyl-2-oxo-[1,4]diazepan-1-yl]-3-naphthalen-1-yl-propionamide,
(R)-2-[4-[3-((R)-(1-Benzyl-2-hydroxy-ethylamino)-propionyl]-3-(3-chloro-phenyl)-5-methyl-2-oxo-[1,4]diazepan-1-yl]-3-naphthalen-1-yl-propionamide,
(R)-2-[4-[3-((S)-1-Benzyl-2-hydroxy-ethylamino)-propionyl]-3-(3-chloro-phenyl)-5-methyl-2-oxo-[1,4]diazepan-1-yl]-3-naphthalen-1-yl-propionamide,
(R)-2-[3-(3-Chloro-phenyl)-4-[3-((1R,2S)-2-hydroxy-1-methyl-2-phenyl-ethylamino)-propionyl]-5-methyl-2-oxo-[1,4]diazepan-1-yl]-3-naphthalen-1-yl-propionamide, and
(R)-2-(3-(3-Chloro-phenyl)-4-{3-[((1S,2R)-2-hydroxy-1-methyl-2-phenyl-ethyl)-methyl-amino]-propionyl}-5-methyl-2-oxo-[1,4]diazepan-1-yl]-3-naphthalen-1-yl-propionamide
(compounds of examples 4 to 8 of TABLE 1 below) are obtained.

### EXAMPLE 9

### 2-[4-(2-Acetylamino-acetyl)-3-(3-chloro-phenyl)-5-methyl-2-oxo-[1,4]diazepan-1-yl]-3-naphthalen-1-yl-propionamide

2-{[3-(3-Chloro-phenyl)-4-amino-methoxy-5-methyl]-2-oxo-[1,4]diazepan-1-yl}-3-naphthalen-1-yl-propionamide is reacted with an excess of acetic anhydride and NaOAc in CH₂Cl₂ at rt. The reaction mixture obtained is diluted with EtAc and extracted with aqueous NaHCO₃ solution and brine. From the mixture obtained solvent is evaporated and the evaporation residue obtained is subjected to chromatography. (R)-2-[(3S,5R)-4-(2-Acetylamino-acetyl)-3-(3-chloro-phenyl)-5-methyl-2-oxo-[1,4]diazepan-1-yl]-3-naphthalen-1-yl-propionamide is obtained.

### EXAMPLE 10

### N-{2-[4-(1-Carbamoyl-2-naphthalen-1-yl-ethyl)-2-(3-chloro-phenyl)-7-methyl-3-oxo-[1,4]diazepan-1-yl]-2-oxo-ethyl}-succinamic acid

A solution of 2-{[3-(3-chloro-phenyl)-4-amino-methoxy-5-methyl]-2-oxo-[1,4]diazepan-1-yl}-3-naphthalen-1-yl-propionamide is treated with an excess of succinic anhydride and N,N-dimethyl-4-aminopyridine in DMF at rt. After 24 hours, the mixture obtained is diluted with EtAc, extracted with 1 N HCl and brine and solvent is evaporated.
N-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethyl}-succinamic acid is obtained.

### EXAMPLE 11

### 2-[4-[2-Amino-acetylamino)-acetyl]-3-(3-chloro-phenyl)-5-methyl-2-oxo-[1,4]diazepan-1-yl]-3-naphthalen-1-yl-propionamide

A solution of 2-{[3-(3-Chloro-phenyl)-4-amino-methoxy-5-methyl]-2-oxo-[1,4]diazepan-1-yl}-3-naphthalen-1-yl-propionamide in DMF, 1.5 equiv. of N-carboxymethyl-succinamic acid, optionally N-Boc protected, and 2 eq. of base (diisopropylethylamine or or DMAP) is stirred with 1.5 equiv. of N-ethyl-N'-(3-dimethylaminopropyl)-carbodiimide for 15 hours. The reaction mixture is diluted with EtAc, extracted with NaHCO₃ solution and 1 N HCl, solvent is evaporated and the evaporation residue obtained is subjected to chromatography. (R)-2-[(3S,5R)-4-[2-Amino-acetylamino)-acetyl]-3-(3-chloro-phenyl)-5-methyl-2-oxo-[1,4]diazepan-1-yl]-3-naphthalen-1-yl-propionamide, optionally in N-Boc-protected form, in the form of a trifluoroacetate is obtained.
De-protection is optionally carried out by dissolving the N-Boc protected (R)-2-[(3S,5R)-4-[2-amino-acetylaminoyacetyl]-3-(3-chloro-phenyl)-5-methyl-2-oxo-[1,4]diazepan-1-yl]-3-naphthalen-1-yl-propionamide in TFA/H₂O 10:1 and stirring at 0° for 4 hours. From the mixture obtained, solvent is evaporated and the residue obtained is lyophilized in the presence of 1 N HCl.
(R)-2-[(3S,5R)-4-[2-Amino-acetylamino)-acetyl]-3-(3-chloro-phenyl)-5-methyl-2-oxo-[1,4]diazepan-1-yl]-3-naphthalen-1-yl-propionamide in the form of a hydrochloride is obtained.

### EXAMPLES 12 and 13

### (2-{2-[4-(1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-ethyl)-carbamic acid tert-butyl ester

### N-{2-[4-(1-Carbamoyl-2-naphthalen-1-yl-ethyl)-2-(3-chloro-phenyl)-7-methyl-3-oxo-[1,4]diazepan-1-yl]-2-oxo-ethyl}-benzamide

Analogously to the method as described in example 11, but using appropriate starting materials, the compounds
(2-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-ethyl)-carbamic acid tert-butyl ester, and
N-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethyl}-benzamide (compounds of examples 12 and 13 in TABLE 1 below) are obtained. In example 12 no de-protection is carried out,

### EXAMPLE 14

### Pyrrolidine-2-carboxylic acid {2-[4-(1-carbamoyl-2-naphthalen-1-yl-ethyl)-2-(3-chlorophenyl)-7-methyl-3-oxo-[1,4]diazepan-1-yl]-2-oxo-ethyl}-amide

A solution of 2-{[3-(3-chloro-phenyl)-4-amino-methoxy-5-methyl]-2-oxo-[1,4]diazepan-1-yl}-3-naphthalen-1-yl-propionamide in CH₂Cl₂, 1.5 equiv. of [(pyrrolidine-2-carbonyl-amino]-acetic acid and 2 eq. polymer supported base (diethylaminomethylpolystyrene) is stirred with 1.5 equiv. EDC for 15 hours. From the mixture obtained a solid is filtered off and the filtrate obtained is subjected to chromatography.
(S)-Pyrrolidine-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide in the form of a trifluoroacetate is obtained.

### EXAMPLES 15 to 34

Analogously to the method as described in example 14, but using appropriate starting materials, compounds of examples 15 to 34 of Table 1 are obtained, namely the compounds (R)-Pyrrolidine-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
2-{2-[-4-(1-Carbamoyl-2-naphthalen-1-yl-ethyl)-2-(3-chloro-phenyl)-7-methyl-3-oxo-[1,4]diazepan-1-yl]-2-oxo-ethylcarbamoyl]-pyrrolidin-1-carboxylic acid tert-butylester, such as (S)-2-{2-[(2S,7R)-4-((R)-1-Carbamoyl-2-naphthalen-1-yl-ethyl-2-(3-chloro-phenyl)-7-methyl-3-oxo-[1,4]diazepan-1-yl]-2-oxo-ethylcarbamoyl]-pyrrolidin-1-carboxylic acid tert-butylester and (R)-2-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethylcarbamoyl]-pyrrolidin-1-carboxylic acid tert-butyl ester,
1-Piperidine-4-yl-pyrrolidine-2-carboxylic acid {2-[4-(1-carbamoyl-2-naphthalen-1-yl-ethyl)-2-(3-chloro-phenyl)-7-methyl-3-oxo-[1,4]diazepan-1-yl]-2-oxo-ethyl}-amide, such as (S)-1-Piperidine-4-yl-pyrrolidine-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
Piperidine-2-carboxylic acid {2-[4-(1-carbamoyl-2-naphthalen-1-yl-ethyl)-2-(3-chloro-phenyl)-7-methyl-3-oxo-[1,4]diazepan-1-yl]-2-oxo-ethyl}-amide, such as (S)-Pipendine-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide, and (R)-Piperidine-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
(2-{2-[4-(1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-piperidine-1-carboxylic acid tert-butyl ester, such as (S)-2-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-piperidine-1-carboxylic acid tert-butyl ester and (R)-2-{2-[(2S,7R)-4-((R)-l-Carbamoyl-2-naphthalen-1-yl-ethyl)-2-(3-chloro-phenyl)-7-methyl-3-oxo-[1,4]diazepan-1-yl]-2-oxo-ethylcarbamoyl}-piperidine-1-carboxylic acid tert-butyl ester,
Piperidine-4-carboxylic acid {2-[4-(1-carbamoyl-2-naphthalen-1-yl-ethyl)-2-(3-chloro-phenyl)-7-methyl-3-oxo-[1,4]diazepan-1-yl]-2-oxo-ethyl}-amide, such as Piperidine-4-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
1-Methyl-piperidine-4-carboxylic acid {2-[4-(1-carbamoyl-2-naphthalen-1-yl-ethyl)-2-(3-chloro-phenyl)-7-methyl-3-oxo-[1,4]diazepan-1-yl]-2-oxo-ethyl}-amide, such as 1-Methyl-piperidine-4-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
2-[4-[2-(3-Amino-propionylamino)-acetyl]-3-(3-chloro-phenyl)-5-methyl-2-oxo-[1,4]diazepan-1-yl]-3-naphthalen-1-yl-propionamide, such as (R)-2--[(3S,5R)-4-[2-(3-Aminopropionylamino)-acetyl]-3-(3-chloro-phenyl)-5-methyl-2-oxo-[1,4]diazepan-1-yl]-3-naphthalen-1-yl-propionamide,
Pyrimidine-2-carboxylic acid {2-[4-(1-carbamoyl-2-naphthalen-1-yl-ethyl)-2-(3-chloro-phenyl)-7-methyl-3-oxo-[1,4]diazepan-1-yl]-2-oxo-ethyl}-amide, such as Pyrimidine-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
2-{3-(3-Chloro-phenyl)-5-methyl-2-oxo-4-[2-(2-piperazine-1-yl-acetylamino)-acetyl]-[1,4]diazepan-1-yl}-3-naphthalen-1-yl-propionamide, such as (R)-2-{(3R,5R)-3-(3-Chlorophenyl)-5-methyl-2-oxo-4-[2-(2-piperazine-1-yl-acetylamino)-acetyl]-[1,4]diazepan-1-yl}-3-naphthalen-1-yl-propionamide and (R)-2-{(3S,5R)-3-(3-Chloro-phenyl)-5-methyl-2-oxo-4-[2-(2-piperazine-1-yl-acetylamino)-acetyl]-[1,4]diazepan-1-yl}-3-naphthalen-1-yl-propionamide,
2-{3-(3-Chloro-phenyl)-5-methyl-2-oxo-4-[2-(2-pyridin-4-yl-acetylamino)-acetyl]-[1,4]diazepan-1-yl}-3-naphthalen-1-yl-propionamide, such as (R)-2-{(3R,5R)-3-(3-Chlorophenyl)-5-methyl-2-oxo-4-[2-(2-pyridin-4-yl-acetylamino)-acetyl]-[1,4]diazepan-1-yl}-3-naphthalen-1-yl-propionamide,
2-{3-(3-Chloro-4-fluoro-phenyl)-5-methyl-2-oxo-4-[2-(2-pyridin-4-yl-acetylamino)-acetyl]-[1,4]diazepan-1-yl}-3-naphthalen-1-yl-propionamide, such as (R)-2-{(3R,5R)-3-(3-Chloro-4-fluoro-phenyl)-5-methyl-2-oxo-4-[2-(2-pyridin-4-yl-acetylamino)-acetyl]-[1,4]diazepan-1-yl}-3-naphthalen-1-yl-propionamide and (R)-2-{(3S,5R)-3-(3-Chloro-4-fluoro-phenyl)-5-methyl-2-oxo-4-[2-(2-pyridin-4-yl-acetylamino)-acetyl]-[1,4]diazepan-1-yl}-3-naphthalen-1-yl-propionamide,
2-{3-(3-Chloro-phenyl)-5-methyl-2-oxo-4-[2-(3-pyridin-3-yl-propionylamino)-acetyl]-[1,4]diazepan-1-yl}-3-naphthalen-1-yl-propionamide, such as (R)-2-{(3S,5R)-3-(3-Chlorophenyl)-5-methyl-2-oxo-4-[2-(3-pyridin-3-yl-propionylamino)-acetyl]-[1,4]diazepan-1-yl}-3-naphthalen-1-yl-propionamide,
2-{3-(3-Chloro-phenyl)-5-methyl-2-oxo-4-[2-(2-pyrimidin-2-yl-acetylamino)-acetyl]-[1,4]diazepan-1-yl}-3-naphthalen-1-yl-propionamide, such as (R)-2-{(3S,5R)-3-(3-Chlorophenyl)-5-methyl-2-oxo-4-[2-(2-pyrimidin-2-yl-acetylamino)-acetyl]-[1,4]diazepan-1-yl}-3-naphthalen-1-yl-propionamide, and
2-{4-[2-(3-Amino-propionylamino)-acetyl]-3-(3-chloro-phenyl)-5-methyl-2-oxo-[1,4]diazepan-1-yl}-3-naphthalen-1-yl-propionamide, such as (R)-2-{(3R,5R)-4-[2-(3-Aminopropionylamino)-acetyl]-3-(3-chloro-phenyl)-5-methyl-2-oxo-[1,4]diazepan-1-yl}-3-naphthalen-1-yl-propionamide.

### EXAMPLE 35

### 2-[4-[3-(3-Amino-propylamino)-propionyl]-3-(3-chloro-phenyl)-5-methyl-2-oxo-[1,4]diazepan-1-yl]-3-naphthal-1-yl-propionamide

A compound of example 56 is treated with Boc-aminopropylamin in THF in the presence of AcOH at rt for 1 day. (R)-2-[4-[3-(3-Amino-propylamino)-propionyl]-3-(3-chloro-phenyl)-5-methyl-2-oxo-[1,4]diazepan-1-yl]-3-naphthal-1-yl-propionamide in Boc-amino protected form is obtained from which 79 mg are dissolved in 3 ml of CH₂Cl₂ and 1.5 ml of 2M HCl in ether are added. The solution obtained is stirred for 2 hours, the mixture obtained is filtered and
(R)-2-[4-[3-(3-Amino-propylamino)-propionyl]-3-(3-chloro-phenyl)-5-methyl-2-oxo-[1,4]diazepan-1-yl]-3-naphthal-1-yl-propionamide in the form of a hydrochloride in solid form is obtained.

### EXAMPLE 36

### ({2-[4-(1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-methyl)-trimethyl-ammonium chloride

The compound of example 11 is dissolved in CH₂Cl₂ in the presence of K₂CO₃ solution and treated with CH₃I under vigorous stirring at rt. From the mixture obtained solvent is partially evaporated and the evaporation residue obtained is diluted with MeOH, the mixture obtained is subjected to a solid phase extraction cartouche, washed with aqueous HCl and eluted with a MeOH/H₂O step-gradient containing HCl.
({2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-methyl)-trimethyl-ammonium chloride is obtained.

### EXAMPLE 37

### 2-{3-(3-Chloro-phenyl)-4-[2-(3-ethyl-ureido)-acetyl]-5-methyl-2-oxo-[1,4]diazepan-1-yl}-3-naphthalen-1-yl-propionamide

A solution of 2-{[3-(3-chloro-phenyl)-5-amino-methoxy]-2-oxo-[1,4]diazepan-1-yl}-3-naphthalen-1-yl-propionamide in CH₂Cl₂ is treated with an excess of ethylisocyanate and DIEA at rt. The reaction mixture obtained is diluted with EtAc, extracted with NaHCO₃ solution + 1 N HCl, solvent is evaporated and the evaporation residue obtained is subjected to chromatography. (R)-2-{(3S,5R)-3-(3-Chloro-phenyl)-4-[2-(3-ethyl-ureido)-acetyl]-5-methyl-2-oxo-[1,4]diazepan-1-yl}-3-naphthalen-1-yl-propionamide is obtained.

### EXAMPLE 38

### 2-[4-{2-[(3-Amino-propionyl)-methyl-amino]-acetyl}-3-(3-chloro-phenyl)-5-methyl-2-oxo-1,4]diazepan-1-yl]-3-naphthalen-1-yl-propionamide

An excess of Boc protected β-alanin (3 equiv.) is pre-activated with CDI (3 equiv.) in DMF at rt for several hours. The pH of the mixture obtained is adjusted to 4 by addition of TFA and the mixture obtained is added to a solution of 2-{[3-(3-chloro-phenyl)-4-amino-methoxy-5-methyl]-2-oxo-[1,4]diazepan-1-yl}-3-naphthalen-1-yl-propionamide (1 equiv.) in DMF. The pH of the reaction mixture is adjusted to 4 with TFA and the mixture obtained is stirred at rt until no further consumption of starting material is detected (thin layer chromatography). The mixture obtained is diluted with EtAc, extracted with phosphate buffer pH=8 or diluted HCl, or both. Solvent is evaporated and the evaporation residue obtained is purified via solid-phase extraction from C-18 cartouches. De-protection is carried out as described in example 11. (R)-2-[(3S,5R)-4-{2-[(3-Amino-propionyl)-methyl-amino]-acetyl}-3-(3-chloro-phenyl)-5-methyl-2-oxo-[1,4]diazepan-1-yl]-3-naphthalen-1-yl-propionamide in the form of a trifluoroacetate is obtained.

### EXAMPLES 39 to 50

Analogously to the method as described for example 38, but using appropriate starting materials, e.g. and using the appropriate carboxylic acid in unprotected form, the following compounds are obtained:
Pyridine-2-carboxylic acid {2-[4-(1-carbamoyl-2-naphthalen-1-yl-ethyl)-2-(3-chloro-phenyl)-7-methyl-3-oxo-[1,4]diazepan-1-yl]-2-oxo-ethyl}-amide, such as Pyridine-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
N-{2-[4-(1-Carbamoyl-2-naphthalen-1-yl-ethyl)-2-(3-chloro-phenyl)-7-methyl-3-oxo-[1,4]diazepan-1-yl]-2-oxo-ethyl}-nicotinamide, such as N-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethyl}-nicotinamide,
N-{2-[4-(1-Carbamoyl-2-naphthalen-1-yl-ethyl)-2-(3-chloro-phenyl)-7-methyl-3-oxo-[1,4]diazepan-1-yl]-2-oxo-ethyl}-isonicotinamide, such as N-{2-[4-((R)-1-Carbamoyl-2-naphthalen-1-yl-ethyl)-2-(3-chloro-phenyl)-7-methyl-3-oxo-[1,4]diazepan-1-yl]-2-oxo-ethyl}-isonicotinamide,
2-{3-(3-Chloro-phenyl)-5-methyl-2-oxo-4-[2-(2-pyridin-2-yl-acetylamino)-acetyl]-[1,4]diazepan-1-yl]-3-naphthalen-1-yl-propionamide, such as (R)-2-((3S,5R)-3-(3-Chlorophenyl)-5-methyl-2-oxo-4-[2-(2-pyridin-2-yl-acetylamino)-acetyl]-[1,4]diazepan-1-yl]-3-naphthalen-1-yl-propionamide,
2-{3-(3-Chloro-phenyl)-5-methyl-2-oxo-4-[2-(2-pyridin-3-yl-acetylamino)-acetyl]-[1,4]diazepan-1-yl]-3-naphthalen-1-yl-propionamide, such as (R)-2-{(3S,5R)-3-(3-Chlorophenyl)-5-methyl-2-oxo-4-[2-(2-pyridin-3-yl-acetylamino)-acetyl]-[1,4]diazepan-1-yl]-3-naphthalen-1-yl-propionamide,
2-{3-(3-chloro-phenyl)-5-methyl-2-oxo-4-[2-(2-pyridin-4-yl-acetylamino)-acetyl]-[1,4]diazepan-1-yl]-3-naphthalen-1-yl-propionamide, such as (R)-2-{(3S,5R)-3-(3-chloro-phenyl)-5-methyl-2-oxo-4-[2-(2-pyridin-4-yl-acetylamino)-acetyl]-[1,4]diazepan-1-yl]-3-naphthalen-1-yl-propionamide,
N-{2-[4-(1-Carbamoyl-2-naphthalen-1-yl-ethyl)-2-(3-chloro-phenyl)-7-methyl-3-oxo-[1,4]diazepan-1-yl]-2-oxo-ethyl]-3-fluoro-benzamide, such as N-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethyl]-3-fluoro-benzamide,
4-Acetylamino-N-{2-[4-(1-carbamoyl-2-naphthalen-1-yl-ethyl)-2-(3-chloro-phenyl)-7-methyl-3-oxo-[1,4]diazepan-1-yl]-2-oxo-ethyl}-benzamide, such as 4-Acetytamino-N-{2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-benzamide,
(3-{2-[4-(1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-phenyl)-carbamic acid tert-butyl ester, such as (3-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethylcarbamoyl}-phenyl)-carbamic acid tert-butyl ester,
Naphthalene-2-carboxylic acid {2-[4-(1-carbamoyl-2-naphthalen-1-yl-ethyl)-2-(3-chlorophenyl)-7-methyl-3-oxo-[1,4]diazepan-1-yl]-2-oxo-ethyl}-amide, such as Naphthalene-2-carboxylic acid {2-[(2S,7R)-4-((R)-1-carbamoyl-2-naphthalen-1-yl-ethyl)-2-(3-chloro-phenyl)-7-methyl-3-oxo-[1,4]diazepan-1-yl]-2-oxo-ethyl}-amide,
2-{3-(3-Chloro-phenyl)-5-methyl-2-oxo-4-[2-(2-quinolin-6-yl-acetylamnino)-acetyl]-[1,4]diazepan-1-yl}-3-naphthalen-1-yl-propionamide, such as (R)-2-{(3S,5R)-3-(3-Chlorophenyl)-5-methyl-2-oxo-4-[2-(2-quinolin-6-yl-acetylamnino)-acetyl]-[1,4]diazepan-1-yl}-3-naphthalen-1-yl-propionamide, and
2-[4-[2-(2-Benzo[1,3]dioxo-5-yl-acetylaminoracetyl]-3-(3-chloro-phenyl)-5-methyl-2-oxo-[1,4]diazepan-1-yl}-3-naphthalen-1-yl-propionamide, such as (R)-2-[(3S,5R)-4-[2-(2-Benzo[1,3]dioxo-5-yl-acetylamino)-acetyl]-3-(3-chloro-phenyl)-5-methyl-2-oxo-[1,4]diazepan-1-yl}-3-naphthalen-1-yl-propionamide: (compounds of examples 39 to 50 in TABLE 1):

### EXAMPLE 51

### 2-(3-(3-Chloro-phenyl)-5-methyl-2-oxo-4-{2-[2-(1-oxy-pyridin-4-yl)-acetylamino]-acetyl}-[1,4]diazepan-1-yl)-3-naphthalen-1-yl-propionamide

The compound of example 44 is dissolved in CH₂Cl₂ and treated with CH₃ReO₃ and H₂O₂ under vigorous stirring at rt for 7 hours. The mixture obtained is diluted with EtAc, extracted with aqueous NaHCO₃ solution and 1 N.HCl, solvent is evaporated and the evaporation residue obtained is subjected to chromatography.
(R)-2-((R)-3-(3-Chloro-phenyl)-5-methyl-2-oxo-4-{2-[2-(1-oxy-pyridin-4-yl)-acetylamino]-acetyl}-[1,4]diazepan-1-yl)-3-naphthalen-1-yl-propionamide is obtained.

### EXAMPLE 52

### 2-{3-(3-Chloro-phenyl)-4-[2-(1,2-dimethyl-1H-imidazole-4-sulfonylamino)-acetyl]-5-methyl-2-oxo-[1,4]diazepan-1-yl}-3-naphthalen-1-yl-propionamide

A 2-phasic solution of 2-{[3-(3-chloro-phenyl)-4-amino-methoxy-5-methyl]-2-oxo-[1,4]diazepan-1-yl}-3-naphthalen-1-yl-propionamide in CH₂Cl₂ and a small amount of aqueous NaHCO₃ is treated with an excess of 1,2-dimethyl-imidazolyl-4-sulfonyl chloride at rt under vigorous stirring. After consumption of starting material (thin layer chromatography), from the mixture obtained the aqueous phase is removed and the CH₂Cl₂-solution is subjected to chromatography.
(R)-2-{(3S,5R)-3-(3-Chloro-phenyl)-4-[2-(1,2-dimethyl-1H-imidazole-4-sulfonylamino)-acetyl]-5-methyl-2-oxo-[1,4]diazepan-1-yl}-3-naphthalen-1-yl-propionamide is obtained.

### EXAMPLE 53

### 2-{3-(3-Chloro-phenyl)-5-methyl-4-[2-(2-morpholin-4-yl-ethanesulfonylamino)-acetyl]-2-oxo-[1,4]diazepan-1-yl}-3-naphthalen-1-yl-propionamide

A solution of 2-{[3-(3-chloro-phenyl)-4-amino-methoxy-5-methyl]-2-oxo-[1,4]diazepan-1-yl}-3-naphthalen-1-yl-propionamide in CH₂Cl₂ is reacted with morpholinoethanesulfonyl chloride in an excess of DIEA at rt. The reaction mixture obtained is extracted with aqueous NaHCO₃ and saturated phosphate buffer pH=4. From the mixture obtained solvent is evaporated and the evaporation residue obtained is subjected to chromatography.
(R)-2-{(R)-3-(3-Chloro-phenyl)-5-methyl-4-[2-(2-morpholin-4-yl-ethanesulfonylamino)-acetyl]-2-oxo-[1,4]diazepan-1-yl}-3-naphthalen-1-yl-propionamide is obtained.

### EXAMPLE 54

### 3-[4-(1-Carbamoyl-2-naphthalen-1-yl-ethyl)-2-(3-chloro-phenyl)-7-ethyl-3-oxo-[1,4]diazepan-1-yl]-3-oxo-propionic acid ethyl ester

80 mg of 2-{[3,5-dimethyl-2-oxo-[1,4]diazepan-1-yl}-3-naphthalen-1-yl-propionamide are dissolved in 2 ml of CH₂Cl₂, 62 µl of DIPEA and 113 µl of mono ethyl malonic acid chloride are added at rt and the mixture obtained is stirred for 4 hours, diluted with CH₂Cl₂, the organic phase obtained is washed with NaHCO₃, dried, solvent is evaporated and the evaporation residue obtained is subjected to chromatography.
3-[4-((R)-1-Carbamoyl-2-naphthalen-1-yl-ethyl)-2-(3-chloro-phenyl)-7-ethyl-3-oxo-[1,4]diazepan-1-yl]-3-oxo-propionic acid ethyl ester is obtained.

### EXAMPLE 55

### 2-(3-(3-Chloro-phenyl)-5-methyl-2-oxo-4-{2-[(pyridin-3-yl-methyl)-carbamoyl]-acetyl}-[1,4]diazepan-1-yl)-3-naphthalen-1-yl-propionamide

N-Pyridin-3-yl-methyl-malonamic acid is prepared in a 2-step reaction by treating chlorocarbonyl-acetic acid methyl ester with excess of pyridine-3-yl-methylamine in CH₂Cl₂ at 0°. After worming up to rt the mixture obtained is diluted with EtAc and extracted with 0.1 N HCl. The aqueous phase obtained is adjusted to pH=8 and extracted with EtAc and the malonic ester amide of 2-(3-(3-Chloro-phenyl)-5-methyl-2-oxo-4-{2-[(pyridin-3-yl-methyl)-carbamoyl]-acetyl}-[1,4]diazepan-1-yl)-3-naphthalen-1-yl-propionamide is obtained, which is saponified with aqueous NaOH at rt. The mixture obtained is brought to pH=7, saturated with Na₂SO₄ and extracted with EtAc/MeOH to give the free acid which is coupled to a compound of formula A5, wherein R₃ is as defined in TABLE 1, EX 55, following reaction procedure A5. (R)-2-((3S,5R)-3-(3-Chloro-phenyl)-5-methyl-2-oxo-4-{2-[(pyridin-3-ylmethyl)-carbamoyl]-acetyl}-[1,4]diazepan-1-yl)-3-naphthalen-1-yl-propionamide is obtained.

### EXAMPLE 56

### 2-[4-Acryloyl-3-(3-chloro-phenyl)-5-methyl-2-oxo-[1,4]diazepan-1-yl]-3-naphthalen-1-yl-propionamide

874 µl of DIPEA and 749 µl of acrylic acid chloride are added to 2 g of 2-{[3,5-dimethyl-2-oxo-[1,4]diazepan-1-yl}-3-naphthalen-1-yl-propionamide in 10 ml of CH₂Cl₂ at rt and the mixture obtained is stirred for 1 hour, diluted with CH₂Cl₂, washed with NaHCO₃, solvent is evaporated and the evaporation residue obtained is subjected to chromatography.

A diastereoisomeric mixture of (R)-2-[4-Acryloyl-3-(3-chloro-phenyl)-5-methyl-2-oxo-[1,4]diazepan-1-yl]-3-naphthalen-1-yl-propionamide is obtained.

### EXAMPLE 57

### 2-[4-(But-2-enoyl)-3-(3-chloro-phenyl)-5-methyl-2-oxo-[1,4]diazepan-1-yl]-3-naphthalen-1-yl-propionamide

Analogously to the method as described for example 56, but using appropriate starting materials the compound (R)-2-[4-((E)-But-2-enoyl)-3-(3-chloro-phenyl)-5-methyl-2-oxo-[1,4]diazepan-1-yl]-3-naphthalen-1-yl-propionamide (Example 57 in Table 1) is obtained.

### EXAMPLE 58

### 2-[3-(3-Chloro-phenyl)-5-methyl-4-(2-morpholin-4-yl-acetyl)-2-oxo-[1,4]diazepan-1-yl]-3-naphthalen-1-yl-propionamide

2-{[3,5-Dimethyl-2-oxo-[1,4]diazepan-1-yl}-3-naphthalen-1-yl-propionamide is reacted with an excess of morpholine in DMF at rt. After 24 hours, the reaction mixture obtained is diluted with EtAc and extracted with brine. Solvent is evaporated and the the evaporation residue obtained is subjected to chromatography.
(R)-2-[(3S,5R)-3-(3-Chloro-phenyl)-5-methyl-4-(2-morpholin-4-yl-acetyl)-2-oxo-[1,4]diazepan-1-yl]-3-naphthalen-1-yl-propionamide is obtained.

### EXAMPLE 59

### 1-{2-[4-(1-Carbamoyl-2-naphthalen-1-yl-ethyl)-2-(3-chloro-phenyl)-7-methyl-3-oxo-[1,4]diazepan-1-yl]-2-oxo-ethyl}-1H-[1,2,3]triazole-4-carboxylic acid

A solution of 2-{[3-(3-chloro-phenyl)-5-azo-methoxy]-2-oxo-[1,4]diazepan-1-yl}-3-naphthalen-1-yl-propionamide and an excess of propinoic acid in tert-BuOH/H₂O 1:1 is stirred with copper powder at rt for 24 hours. Copper powder is removed, e.g. by centrifugation, and the filtrate obtained is worked-up by extraction and subjected to chromatography.
1-{2-[(2S,7R)-4-((R)-1-Carbamoyl-2-naphthalen-1-yl-ethyl)-2-(3-chloro-phenyl)-7-methyl-3-oxo-[1,4]diazepan-1-yl]-2-oxo-ethyl}-1H-[1,2,3]triazole-4-carboxylic acid is obtained.

### EXAMPLES 60 to 63

### Analogously to the method as described in example 59, but using appropriate starting materials, the compounds

2-(3-(3-Chloro-phenyl)-4-{2-[4-(2-hydroxy-ethyl)-[1,2,3]triazol-1-yl]-acetyl}-5-methyl-2-oxo-[1,4]diazepan-1-yl)-3-naphtha!en-1-yl-propionamide, such as (R)-2-((3S,5R)-3-(3-Chlorophenyl)-4-{2-[4-(2-hydroxy-ethyl)-[1,2,3]triazol-1-yl]-acetyl}-5-methyl-2-oxo-[1,4]diazepan-1-yl)-3-naphthalen-1-yl-propionamide,
2-{3-(3-Chloro-phenyl)-4-{2-[4-dimethylaminomethyl-[1,2,3]triazol-1-yl]-acetyl}-5-methyl-2-oxo-[1,4]diazepan-1-yl)-3-naphthalen-1-yl-propionamide, such as (R)-2-{(3S,5R)-3-(3-Chloro-phenyl)-4-{2-[4-dimethylaminomethyl-[1,2,3]triazol-1-yl]-acetyl}-5-methyl-2-oxo-[1,4]diazepan-1-yl)-3-naphthalen-1-yl-propionamide,
2-{3-(3-Chloro-phenyl)-5-methyl-2-oxo-4-[2-(4-pyridin-3-yl-[1,2,3]triazol-1-yl]-acetyl}-[1,4]diazepan-1-yl}-3-naphthalen-1-yl-propionamide such as (R)-2-{(3S,5R)-3-(3-Chlorophenyl)-5-methyl-2-oxo-4-[2-(4-pyridin-3-yl-[1,2,3]triazol-1-yl]-acetyl}-[1,4]diazepan-1-yl}-3-naphthalen-1-yl-propionamide, and
2-{3-(3-Chloro-phenyl)-5-methyl-2-oxo-4-[2-(4-pyridin-2-yl-[1,2,3]triazol-1-yl]-acetyl}-[1,4]diazepan-1-yl}-3-naphthalen-1-yl-propionamide, such as (R)-2-{(3S,5R)-3-(3-Chlorophenyl)-5-methyl-2-oxo-4-[2-(4-pyridin-2-yl-[1,2,3]triazol-1-yl]-acetyl}-[1,4]diazepan-1-yl}-3-naphthalen-1-yl-propionamide, (compound of examples 60 to 63 in TABLE 1) are obtained.

### Example 64 and 65

### Analogously to a method as described above, but using appropriate starting materials, the compounds

2-[3-(3-Chloro-phenyl)- 4-(2-1H-imidazol-4-yl-acetyl}-5-methyl-2-oxo-[1,4]diazepan-1-yl]-3-naphthalen-1-yl-propionamide, such as (R)-2-[(3S,5R)-3-(3-Chloro-phenyl)- 4-(2-1H-imidazol-4-yl-acetyl}-5-methyl-2-oxo-[1,4]diazepan-1-yl]-3-naphthalen-1-yl-propionamide, and Piperidine-4-carboxylic acid {2-[4-(1-carbamoyl-2-naphthalen-1-yl-ethyl)-2-(3-chloro-4-fluorophenyl)-7-methyl-3-oxo-[1,4]diazepan-1-yl]-2-oxo-ethyl}-amide, such as Piperidine-4-carboxylic acid {2-[(2S,7R)-4-((R)-1-carbamoy!-2-naphthalen-1-yl-ethyl)-2-(3-chloro-4-fluorophenyl)-7-methyl-3-oxo-[1,4]diazepan-1-yl]-2-oxo-ethyl}-amide (Examples 64 and 65 in TABLE 1) are obtained.

In TABLE 1 below there are set out compounds of formula 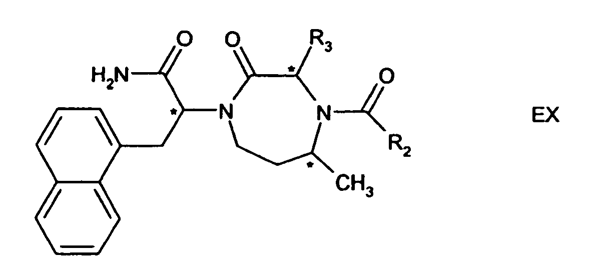 e.g. including compounds of formula 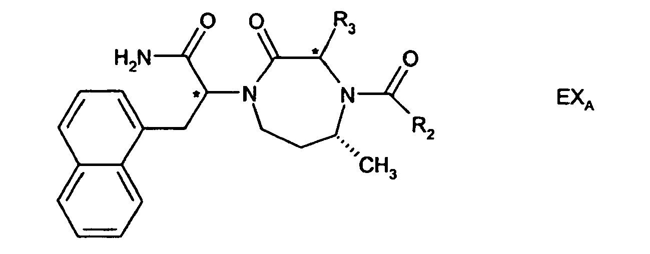 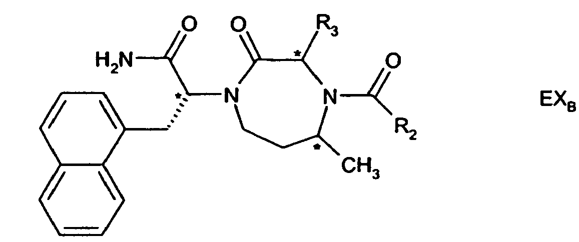 and 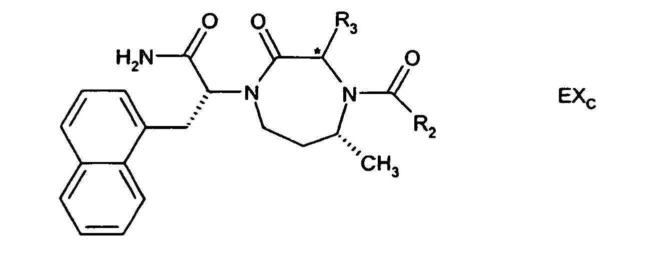 wherein R₂ and R₃ are as described in in TABLE 1 below:

**TABLE 1**

| **EX** | **R₂** | **R₃** | **DATA MS and R_{f}** |
|---|---|---|---|
| 1 | -CH₂-NH-CH₃ | 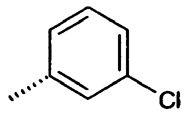 | 507.3 0.65 (KG-60), CH₃OH/H₂O 80:20 TFA |
| 2 | 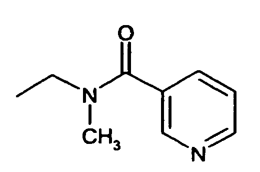 | 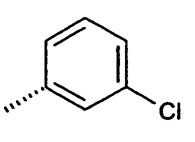 | 634.22 0.23 (KG-60), Tol/i-PrOH 1:1 |
| 3 | -CH₂-CH₂-N(n-Bu)₂ | 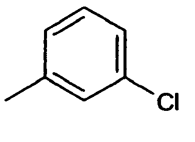 | 619 0.29 (Silicagel) CH₂Cl₂/CH₃OH 9:1 |
| 4 | 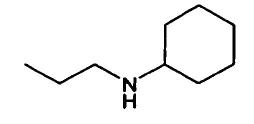 | 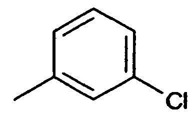 | 589 0.6 (Silicagel) CH₂Cl₂/CH₃OH/NH₄OH 9:1:0.05) |
| 5 | 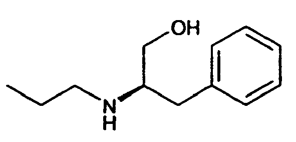 | 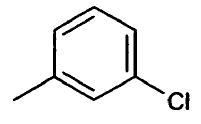 | 663 0.33 (Silicagel) CH₂Cl₂/CH₃OH 9:1 |
| 6 | 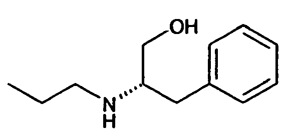 | 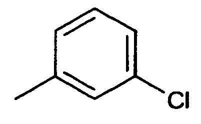 | 641 0.3 (Silicagel) CH₂Cl₂/CH₃OH 9:1 |
| 7 | 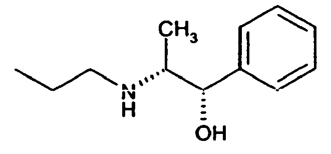 | 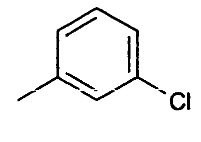 | 641 0.3 (Silicagel) CH₂Cl₂/CH₃OH 9:1 |
| 8 | 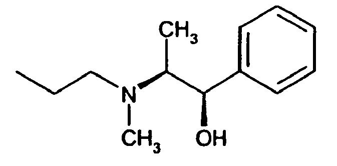 | 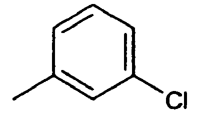 | 677 0.33, (Silicagel) CH₂Cl₂/CH₃OH 9:1 |
| 9 | -CH₂-NH-CO-CH₃ | 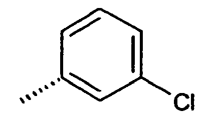 | 557.15 0.35 (KG-60), Tol/i-PrOH 1:1 |
| 10 | -CH₂-NH-CO-(CH₂)₂-COOH | 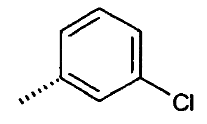 | 591.4 0.53 (KG-60), T/i-PrOH 1:1 |
| 11 | -CH₂-NH-CO-CH₂-NH₂ | 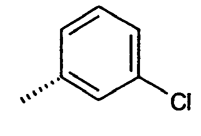 | 550.2 0.32 (KG-60), BuOH/CH₃OH/NaCl/AcOH 10:5:2:1 |
| 12 | -CH₂-NH-CO-(CH₂)₂-NH-Boc | 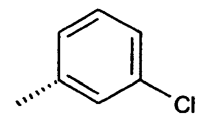 | 686.3 0.29 (KG-60). Tol/i-PrOH 4:1 |
| 13 | 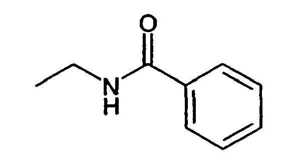 | 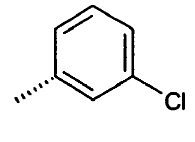 | 619.2 0.49 (KG-60), Tol/i-PrOH 4:1 |
| 14 | 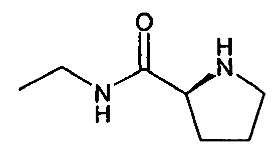 | 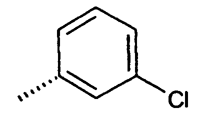 | 590.27 0.26 (KG-60), BuOH/CH₃OH/NaCl/AcOH 10:5:2:1 |
| 15 | 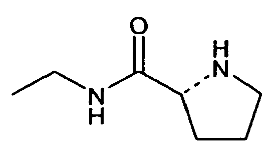 | 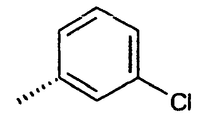 | 590.28 0.26 (KG-60), BuOH/CH₃OH/NaCl/AcOH 10:5:2:1 |
| 16 | 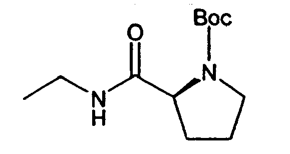 | 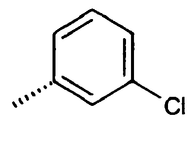 | 712.3 0.45 (KG-60), Tol/i-PrOH 4:1 |
| 17 | 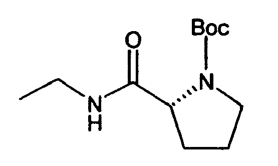 | 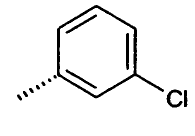 | 712.34 0.45 (KG-60). Tol/i-PrOH 4:1 |
| 18 | 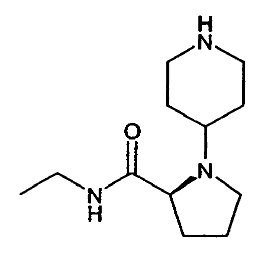 | 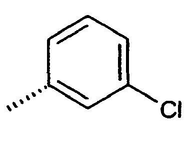 | 673.41 0.09 n(KG-60), BuOH/CH₃OH/NaCl/AcOH 10:5:2:1 |
| 19 | 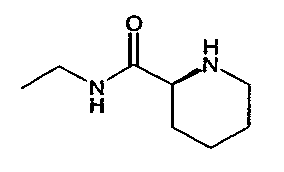 | 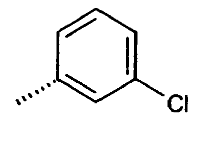 | 604.29 0.30 (KG-60) BuOH/CH₃OH/NaCl/AcOH 10:5:2:1 |
| 20 | 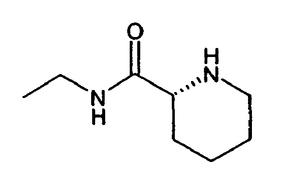 | 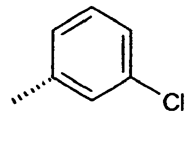 | 604.33 0.30 (KG-60), BuOH/CH₃OH/NaCl/AcOH 10:5:2:1 |
| 21 | 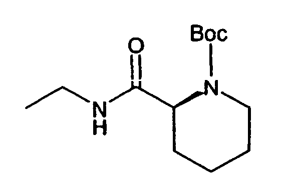 | 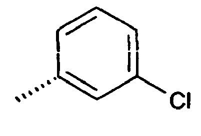 | 726.33 0.50 (KG-60), Tol/i-PrOH 4:1 |
| 22 | 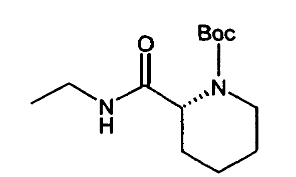 | 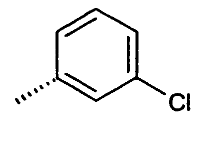 | 726.32 0.50 (KG-60). Tol/i-PrOH 4:1 |
| 23 | 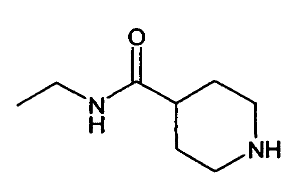 | 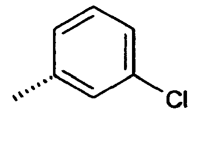 | 604.29 0.70 (RP-8), CH₃OH/H₂O 80:20 TFA |
| 24 | 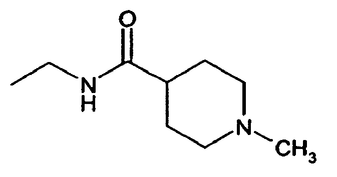 | 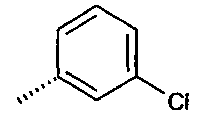 | 618.3 0.70 (RP-8), CH₃OH/H₂O 80:20 TFA |
| 25 | -CH₂-NH-CO-(CH₂)₂-NH₂ | 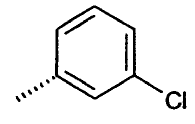 | 564.3 0.30 (RP-8), CH₃OH/H₂O 80:20 NH₃ |
| 26 | 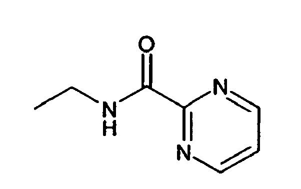 | 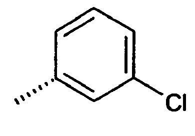 | 621.1 0.59 (RP-8), CH₃OH/H₂O 80:20 TFA |
| 27 | 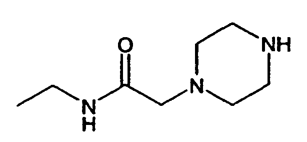 | 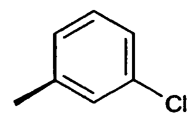 | 641.31 0.60 (RP-8), CH₃OH/H₂O 80:20 TFA |
| 28 | 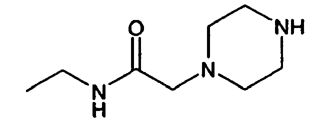 | 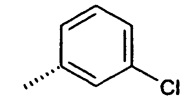 | 619.28 0.65 (RP-8), CH₃OH/H₂O 80:20 TFA |
| 29 | 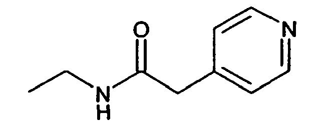 | 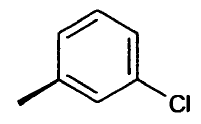 | 634.08 0.14 (KG-60), Tol/i-PrOH 1:1 |
| 30 | 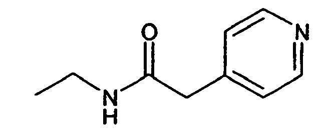 | 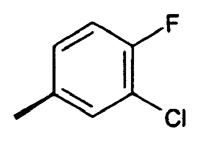 | 652.11 0.30 (KG-60), T/CH₃OH 5:1 |
| 31 | 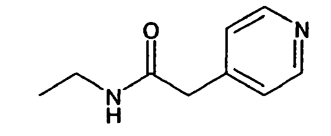 | 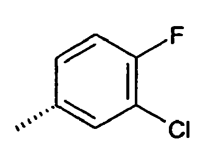 | 652.04 0.35 (KG-60), T/CH₃OH 5:1 |
| 32 | 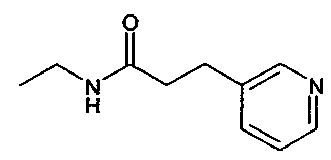 | 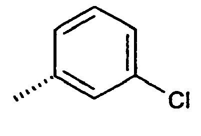 | 648.17 0.24 (KG-60). Tol/i-PrOH 1:1 |
| 33 | 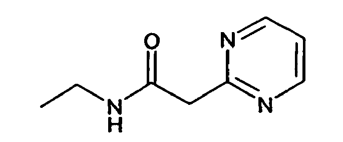 | 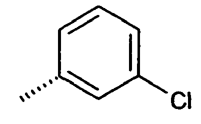 | 635.12 0.62 (RP-8), CH₃OH/H₂O 80:20 TFA |
| 34 | -CH₂-NH-CO-(CH₂)₂-NH₂ | 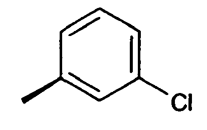 | 586.23 0.60 (RP-8), CH₃OH/H₂O 80:20 TFA |
| 35 | -(CH₂)₂-NH-(CH₂)₃-NH₂ | 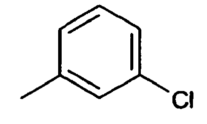 | 564 0.31, (Silicagel) CH₂Cl₂/CH₃OH 9:1 |
| 36 | -CH₂-NH-CO-CH₂-N⁺(CH₃)₃ Cl⁻ | 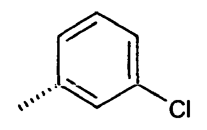 | 592.3 0.32 (KG-60), BuOH/CH₃OH/NaCl/AcOH 10:5:2:1 |
| 37 | -CH₂-NH-CO-NH-CH₂-CH₃ | 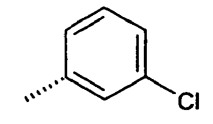 | 586.2 0.38 (KG-60), CH₂Cl₂/i-PrOH 9:1 |
| 38 | -CH₂-N(CH₃)-CO-(CH₂)₂-NH₂ | 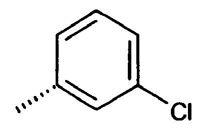 | 578.18 0.40 (KG-60), BuOH/CH₃OH/NaCl/AcOH 10:5:2:1 |
| 39 | 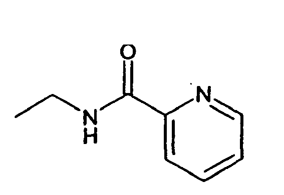 | 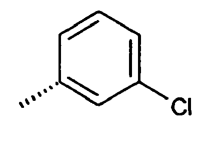 | 620.3 0.68 (KG-60), Tol/i-PrOH 1:1 |
| 40 | 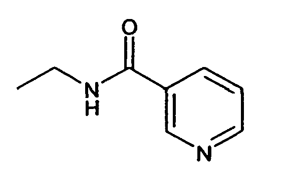 | 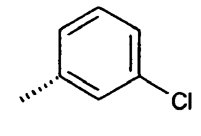 | 620.2 0.38 (KG-60), Tol/i-PrOH 1:1 |
| 41 | 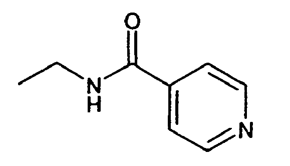 | 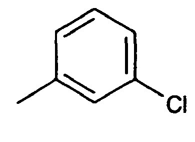 | 620.2 0.33; 0.37 (KG-60) T/i-PrOH 1:1 |
| 42 | 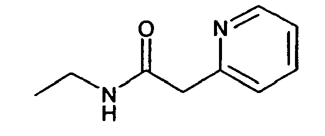 | 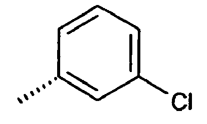 | 634.3 0.44 (KG-60), T/i-PrOH 1:1 |
| 43 | 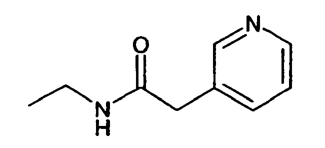 | 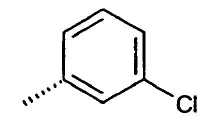 | 634.3 0.22 (KG-60), Tol/i-PrOH 1:1 |
| 44 | 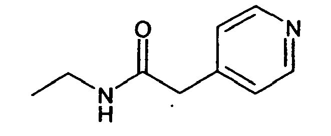 | 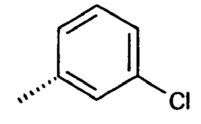 | 634.4 0.25 (KG-60), Tol/iPrOH 1:1v |
| 45 | 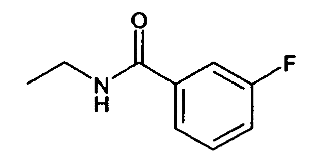 | 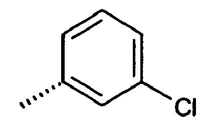 | 637.2 0.71 (KG-60), Tol/i-PrOH 1:1 |
| 46 | 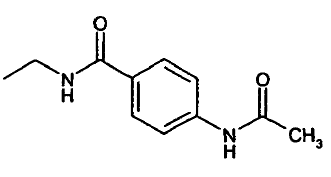 | 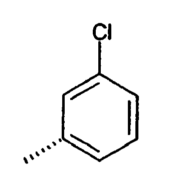 | 676.3 0.33 (KG-60), Tol/i-PrOH 1:1 |
| 47 | 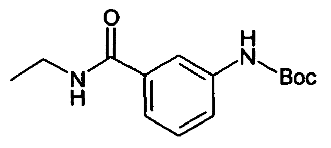 | 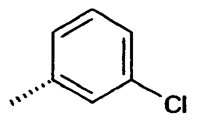 | 734.3 0.78 (KG-60), Tol/i-PrOH 1:1 |
| 48 | 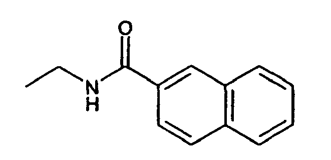 | 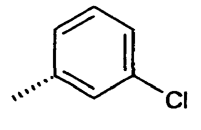 | 669.2 0.43 (KG-60), Tol/i-PrOH 1:1 |
| 49 | 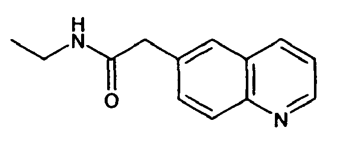 | 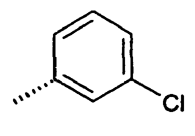 | 696.44 0.42 (KG-60), Tol/i-PrOH 1:1 |
| 50 | 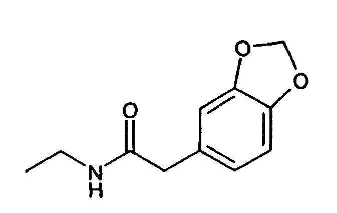 | 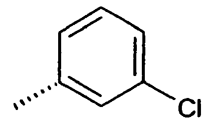 | 677.34 0.35 (KG-60), Tol/i-PrOH 4:1 |
| 51 | 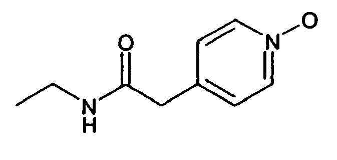 | 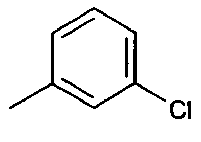 | 650.08 0.43(KG-60), BuOH/CH₃OH/NaCl/AcOH 10:5:2:1 |
| 52 | 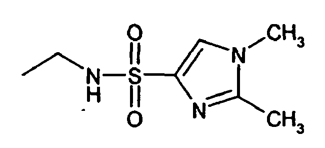 | 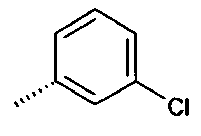 | 673.22 0.25 (KG-60), Tol/i-PrOH 1:1 |
| 53 | 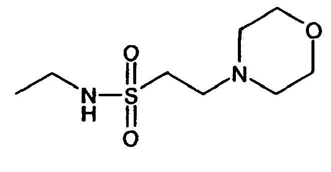 | 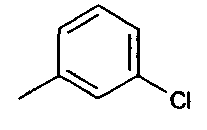 | 692.14 0.45 (KG-60), BuOH/CH₃OH/NaCUAcOH 10:5:2:1 |
| 54 | 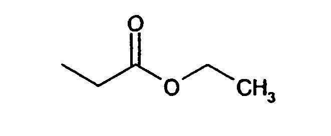 | 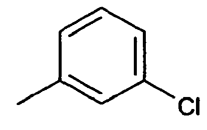 | 572 0.45, CH₂Cl₂/CH₃OH 95:5 |
| 55 | 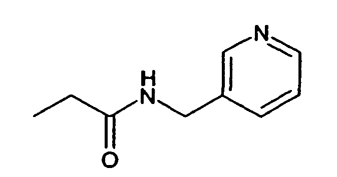 | 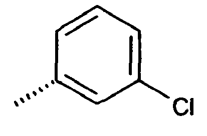 | 634.1 0.59 (RP-8), CH₃OH/H₂O 80:20 NH₃ |
| 56 | -CH=CH | 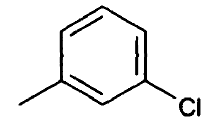 | 512 0.7, (Silicagel) CH₂Cl₂/CH₃OH 95:5 |
| 57 | -CH=CH-CH₃ | 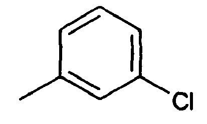 | 526 0.5, (Silicagel) CH₂Cl₂/CH₃OH 95:5 |
| 58 | 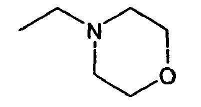 | 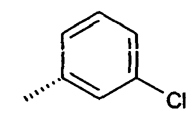 | 585.2 0.38 (KG-60), Tol/i-PrOH 4:1 |
| 59 | 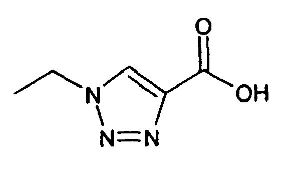 | 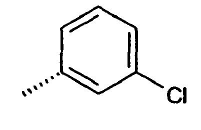 | 633.16 0.56 (KG-60) BuOH/ CH₃OH/NaCl/AcOH 10:5:2:1 |
| 60 | 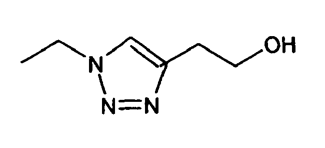 | 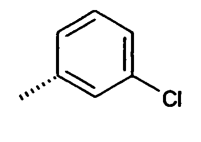 | 611.22 0.28 (KG-60) Tol/i-PrOH 1:1 |
| 61 | 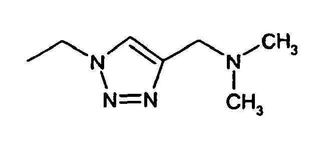 | 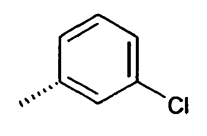 | 624.24 0.24 (KG-60) CH₂Cl₂/CH₃OH 1:1 |
| 62 | 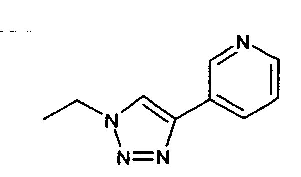 | 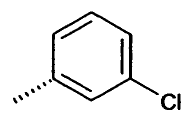 | 644.22 0.53 (RP-8) CH₃OH/H₂O 80:20 TFA |
| 63 | 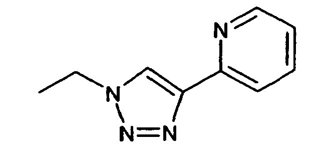 | 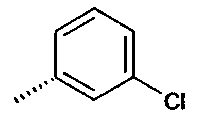 | 644.22 0.54 (RP-8) CH₃OH/H₂O 80:20 TFA |
| 64 | 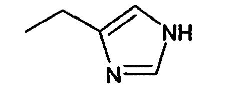 | 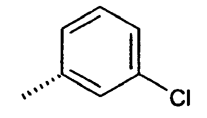 | 554.18 0.66 (RP-8) CH₃OH/H₂O 80:20 TFA |
| 65 | 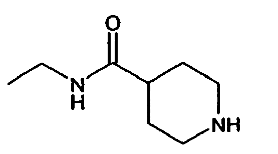 | 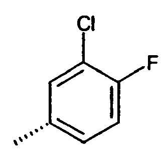 | 644.24 0.40 (KG-60) BuOH/CH₃OH/NaCl/AcOH 10:5:2:1 |

In TABLE 1 the compounds of examples 1, 11, 12, 14, 15, 18, 19, 20, 23, 24, 27, 28, 34 and 38 are obtained in the form of a trifluoroacetate, the compounds of examples 2 to 8, 25, 26, 30 to 33, 35, 49, 60 to 65 are obtained in the form of a hydrochloride, the compound of example 35 is obtained in the form of a trimethylammoniumchloride and all other compounds are obtained in the form of the free base.

Under "DATA" in TABLE 1, MS (mass spectroscopy) data are ESI data and the indicated R_{f} values of thin layer chromatography are given for the compounds together with the thin layer material and the solvent system used for chromatography.

### Preparation of intermediates (starting materials)

### Preparation of naphythylalanine amide (Compound of formula A1)

**Naphthalene-1-carboxylic** acid is dissolved in dry THE and 5 equiv. of borane dimethylsulfide complex are added. The mixture obtained is stirred at rt, diluted with EtAc, washed with 1 N HCl and 5% NaHCO₃ solution, dried and solvent is evaporated. (Naphthalene-1-yl)-methanol is obtained, which is dissolved in CH₂Cl₂. To the solution obtained 1.5 equiv. of Dess-Martin reagent are added at rt. The mixture obtained is diluted with EtAc, extracted with 1 N HCl and 5% NaHCO₃-solution, dried and solvent is evaporated. Naphthalene-1-carboxaldehyde is obtained and dissolved with 1 equiv. of racemic-Boc-α-phosphonoglycine trimethylester in CH₂Cl₂ and 1.1 equiv. of DBU are added. The mixture obtained is stirred at rt and treated in sequence with 1 N HCl and 5% NaHCO₃ solution. The phases obtained are separated, the organic phase obtained is dried and solvent is evaporated. 2-Boc-amino-3-(naphthalene-1-yl)-acrylic acid methyl ester (cis/trans mixture) is obtained, is dissolved in MeOH/H₂O at pH 6.5 (phosphate buffer) and 20 w/w% of 10% Pd/C are added. The mixture obtained is hydrogenated at rt and 50 bar, the catalyst is filtered off and from the filtrate obtained solvent is evaporated. Racemic naphythylalanine methylester is obtained, dissolved in methanolic NH₃ and stirred. The mixture obtained is subjected to extractive work up. Racemic naphythylalanine amide is obtained.

## Claims

1. A compound of formula 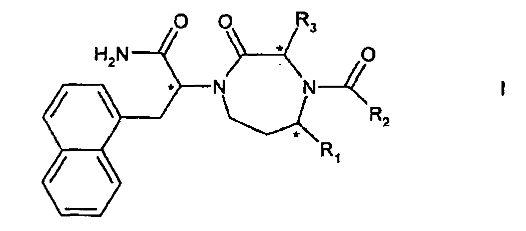 wherein
R₁ is methyl;
R₂ is pyridinylmethyl-aminocarbonyl-methyl, optionally in the form of an N-oxide; and
R₃ is (C₆₋₁₈)aryl substituted by (C₁₋₄)alkyl, (C₁₋₄)alkoxy halo(C₁₋₆)alkyl, halo(C₁₋₆)alkoxy, cyano,carboxy, hydroxy, amino, (C₁₋₆)alkyl- and (C₁₋₆)dialkylamino, (C₁₋₆)alkyclarbonylamino, (C₁₋₈)alkoxycarbonyl-amino, (C₁₋₈)alkoxycarbonyl, heterocyclyl, or halogen.

2. A compound according to claim 1, wherein R₁ and R₂ are as defined in claim 1 and R₃ is phenyl substituted by one or more halogen.

3. A compound according to any one of claims 1 or 2, selected from the group consisting of
2-{3-(3-Chloro-phenyl)-5-methyl-2-oxo-4-[2-(2-pyridin-4-yl-acetylamino)-acetyl]-[1,4]diazepan-1-yl}-3-naphthalen-1-yl-propionamide,
2-{3-(3-Chloro-4-fluoro-phenyl)-5-methyl-2-oxo-4-[2-(2-pyridin-4-yl-acetylamino)-acetyl]-[1,4]diazepan-1-yl}-3-naphthalen-1-yl-propionamide,
2-{3-(3-Chloro-phenyl)-5-methyl-2-oxo-4-[2-(2-pyridin-2-yl-acetylamino)-acetyl]-[1,4]diazepan-1-yl]-3-naphthalen-1-yl-propionamide,
2-{3-(3-Chloro-phenyl)-5-methyl-2-oxo-4-[2-(2-pyridin-3-yl-acetylamino)-acetyl]-[1,4]diazepan-1-yl]-3-naphthalen-1-yl-propionamide,
2-(3-(3-Chloro-phenyl)-5-methyl-2-oxo-4-{2-[2-(1-oxy-pyridin-4-yl)-acetylamino]-acetyl}-[1,4]diazepan-1-yl)-3-naphthalen-1-yl-propionamide and
2-(3-(3-Chloro-phenyl)-5-methyl-2-oxo-4-{2-[(pyridin-3-yl-methyl)-carbamoyl]-acetyl}-[1,4]diazepan-1-yl)-3-naphthalen-1-yl-propionamide.

4. A compound of any one of claims 1 to 3 in the form of a salt.

5. A compound of any one of claims 1 to 4 for use as a pharmaceutical.

6. A pharmaceutical composition comprising a compound of any one of claims 1 to 4 in association with at least one pharmaceutical excipient.

7. Use of a compound of any one of claims 1 to 4 for the manufacture or a medicament for the treatment of disorders which are mediated by LFA-1 activity; said disorder being selected from the group consisting of inflammatory disorders, allergic disorders, and autoimmune disorders.

8. A combination of a compound of any one of claims 1 to 4 with at least one second drug substance.

9. A compound of of any one of claims 1 to 5 in combination with at least one second drug substance for use in treating a disorder mediated by LFA-1 activity; said disorder being selegted from the group consisting of inflammatory disorders, allergic disorders, and autoimmune disorders.

## Patentansprüche

1. Verbindung der Formel 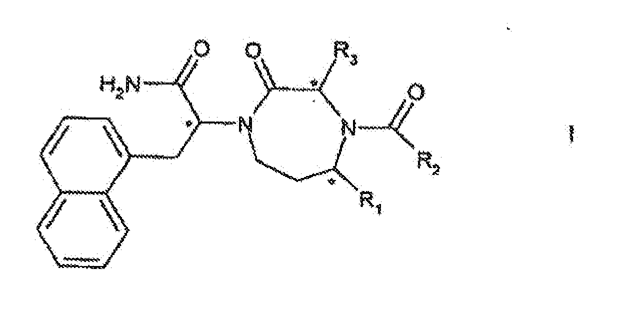 worin
R₁ für Methyl steht;
R₂ für Pyridingylmethyl aminocarbonylmethyl, gegebenenfalls in Form eines N-Oxids, steht und
R₃ für (C₆₋₁₈)-Aryl steht, das durch (C₁₋₄)-Alkyl, (C₁₋₄)-Alkoxy, Halogen-(C₁₋₆)-alkyl, Halogen-(C₁₋₆)-alkoxy, Cyane, Carcboxy, Hydroxy, Amino, (C₁₋₆)-Alkyl- und (C₁₋₆)-Dialkylamin, (C₁₋₆)-Alkylcarbonylamino, (C₁₋₆)-Alkylcarbonylamino, (C₁₋₈)-Alkoxycarbonyl, Heterocyclyl oder Halogen substituiert ist.

2. Verbindung nach Anspruch 1, worin R₁ und R₂ die in Anspruch 1 angegeben Bedeutung besitzen und R₃ für Phenyl steht, das durch ein oder mehrere Halogene substituiert ist.

3. Verbindung nach einem der Ansprüche 1 oder 2, ausgewählt aus der Gruppe bestehend aus
2-{3-(3-Chlorphenyl)-5-methyl-2-oxo-4-[2-(2-pyridin-4-ylacetylamino)acetyl][1,4]diazepan-1-yl}-3-3-naphthalin-1-ylpropionamind.
2-{3-(3-Chlor-4-fluorphenyl)-5-methyl-2-oxo-4-[2-(2-pyridin-4-ylacetylamino)acety][1,4]diazepan-1-yl}-3-naphthalin-1-ylpropionamid,
2-{3-(3-Chlorphenyl)-5-methyl-2-oxo-4-[2-(2-pyridin-4-ylacetylmnino)acetyl][1,4]diazepan-1-yl}-3-naphthalin-1-ylpropionamid,
2-{3-(3-Chlorphenyl)-5-methyl-2-oxo-4-[2-(2-pyridin-3-ylacetylamino)acetyl][1,4]diazepan-1-yl}-3--naphthalin-1-ylpropionamid,
2-(3-(3-Chlorphenyl)-5-methyl-2-oxo-4-{2-[2-(1-oxypyridin-4-yl)acetylamino]acetyl}1,4]diazepan-1-yl)-3-naphthalin-1-ylpropionamid und
2-(3-(3-Chlorphenyl)-5-methyl-2-oxo-4-{2-[(pyridin-3-ylmethyl)carbamoyl]acetyl}[1,4]diazepan-1-yl)-3-naphthalin-1-ylpropionamid.

4. Verbindung nach einem der Ansprüche 1 bis 3 in Form eines Salzes.

5. Verbindung nach einem der Ansprüche 1 bis 4 zur Verwendung als Pharmazeutikum.

6. Pharmazeutische Zusammensetzung, enthaltend eine Verbindung nach einem der Ansprüche 1 bis 4 zusammen mit mindestens einem pharmazeutischen Hilfsstoff.

7. Verwendung einer Verbindung nach einem der Ansprüche 1 bis 4 zur Herstellung eines Arzneimittels zur Behandlung von Störungen, die durch LFA-1-Altivität vermittelt werden; wobei die Störung aus der Gruppe bestehend aus entzündlichen Störungen, allergischen Störungen und Autoimmunstörungen ausgewählt ist.

8. Kombination einer Verbindung nach einem der Anspruche 1. bis 4 mit mindestens einem zweiten Arzneistoff.

9. Verbindung nach einem der Ansprüche 1 bis 5 in Kombination mit mindestens einem zweiten Arzneistoff zur Verwendung bei der Behandlung einer durch LFA-1-Aktivität vermittelten Störung; wobei die Störung aus der Gruppe bestehend aus entzündlichen Störungen, allergischen Störungen und Autoimmunstörungen ausgewählt ist.

## Revendications

1. composé de formule 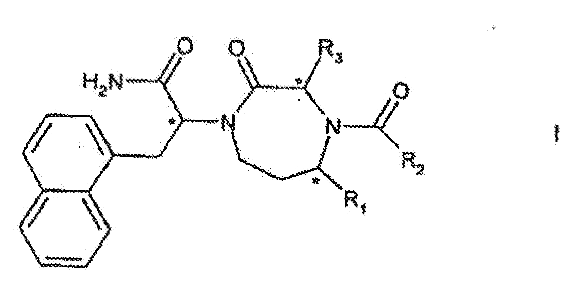 dans laquelle
R₁ est méthyle;
R₂ est pyridinylméthyl-aminocarbonyl-méthyle, éventuellement sous forme d'un N-oxyde ; et
R₃ est (C₆₋₁₈)aryle substitué par (C₁₋₄)alkyle, (C₁₋₄)alcoxy halogéno(C₁₋₆)alkyle, halogéne(C₁₋₆)alcoxy, cyano, carboxy, hydroxy, amino, (C₁₋₆)alkyl- et (C₁₋₆)dialkylamino, (C₁₋₆)alkylcarbonylamino, (C₁₋₆)alcoxycarbonylamino, (C₁₋₈)alcoxycarbonyle, hetérocyelyle, ou halogène.

2. Composé selon la revendication 1, **caractérisé en ce que** R₁ et R₂ sont tels que définis dans la revendication 1 et R₃ est phényle substitué par un ou plusieurs halogènes.

3. Composé selon l'une ou l'autre des revendications 1 et 2, choisi dans le groupe constitué de
2-{3-(3-chloro-phényl)-5-méthyl-2-oxo-4-[2-(2-pyridin-4-yl-acétylamino)-acétyl]-[1,4]diazépan-1-yl}-3-naphtalén-1-yl-propionamide,
2-{3-(3-chloro-4-fluoro-phényl)-5-2-oxo-4-[2-(2-pyridin-4-yl-acétylamino)-acétyl]-[1,4]diazépan-1-yl}-3-naphtalén-1-yl-propionamide,
2-{3-(3-chloro-phényl)-5-méthyl-2-oxo-4-[2-(2-pyridin-2-yl-acétylamino)-acétyl]-[1,4]diazépan-1-yl}-3-naphtalén-1-yl-propionamide,
2-{3-(3-chloroényl)-5-méthl-2-oxo-4-[2-(2-pyridin-3-yl-acétylamino)-acétyl]-[1,4]diazépan-1-yl}-3-naphtalén-1-yl-propionamide,
2-(3-(3-chloro-phényl)-5-méthyl-2-oxo-4-{2-[2-(1-oxy-pyridin-4-yl)-acétylamino]-acétyl}-[1,4]diazépan-1-yl)-3-naphtalén-1-yl-propionamide et
2-(3-(3--chloro-phényl)-5-méthyl-2-oxo-4-{2-[(pyridin-3-yl-méthyl)-carbamoyl]-acétyl}-[1,4]diazépan-1-yl)-3-naphtalén-l-yl-propionamide.

4. Composé selon l'une quelconque des revendications 1 à 3, soues forme d'un sel.

5. Composé selon l'une quelconque des revendications 1. à 4, destiné à être utilisé comme produit pharmaceutique.

6. Composition pharmaceutique comprenant un composé selon l'une quelconque des revendications 1 à 4, en association avec au moins un excipient pharmaceutique.

7. Utilisation d'un composé selon l'une quelconque des revendications 1 à 4, pour la fabrication d'un médicament destiné au traitement des troubles qui sont médiés par l'activité du LFA-1 ; ledit trouble étant choisi dans le groupe constitué de troubles inflammatoires, de troubles allergiques et de troubles auto-immuns.

8. Combinaison d'un composé selon l'une quelconque des revendications 1 à 4, avec au moins une deuxième substance médicamenteuse.

9. Composé selon l'une quelconque des revendications 1 à 5, en combinaison aven au moins une deuxième substance médicamenteuse, destiné à être utilisé dans le traitement d'un trouble médié par l'activité du LFA-1; ledit trouble étant choisi dans le groupe constitué de troubles inflammatoires, de troubles allergiques et de troublés auto-immuns.
